# FASCICULE DE BREVET EUROPEEN

(11) **EP 0 732 328 B1**
(45) Date de publication et mention de la délivrance du brevet: **09.09.1998**
(21) Numéro de dépôt: 96400450.1
(22) Date de dépôt: 01.03.1996
(51) Int. Cl.: C07D 311/58, C07D 335/06, C07D 215/02, C07D 405/04, C07D 409/04, C07D 407/04, C07C 69/92, A61K 31/35, A61K 7/48

(54) **Composés hétérocycliques aromatiques, compositions pharmaceutiques et cosmétiques les contenant et utilisations**
Heterocyclische, aromatische Verbindungen, pharmazeutische und kosmetische Zusammensetzungen,die sie enthalten, und Verwendungen.
Aromatic heterocyclic compounds, pharmaceutical and cosmetic compositions containing them and their uses

(30) Priorité: 14.03.1995 FR 9502926
(43) Date de publication de la demande: 18.09.1996
(73) Titulaire: CENTRE INTERNATIONAL DE RECHERCHES DERMATOLOGIQUES GALDERMA (C.I.R.D. GALDERMA), F-06560 Valbonne (FR)
(72) Inventeur: Charpentier, Bruno, F-06410 Biot (FR); Nedoncelle, Philippe, F-06130 Grasse (FR); Diaz, Philippe, F-06200 Nice (FR)
(74) Mandataire: Tezier Herman, Béatrice

(56) Documents cités:
- EP-A- 0 025 598
- EP-A- 0 199 636
- EP-A- 0 267 155
- WO-A-92/21663
- DE-A- 2 006 505
- GB-A- 2 164 648
- J. AGRIC. FOOD CHEM., vol. 25, no. 4, 1977, pages 723-726, XP000574803 L. JURD ET AL:
- TETRAHEDRON, vol. 42, no. 3, 1986, pages 863-876, XP000196184 S. RAMAKANTII ET AL:
- MOKUZAI GAKKAISHI, vol. 33, no. 3, 1987, pages 234-238, XP000196186 T. MITSUNAGA ET AL:
- J. CHEM. RES., SYNOP., no. 10, 1985, page 305 XP000196194 T.C.A. AFONYA ET AL:
- CHEMICAL ABSTRACTS, vol. 104, no. 12, 1986 Columbus, Ohio, US; abstract no. 95475m, XP002007512 & JP-A-60 178 815 (INSTITUTE OF PHYSICAL AND CHEMICAL RESEARCH)
- SYNTHESIS, no. 2, 1988, pages 155-157, XP000196183 A. ARNOLDI ET AL:
- J. STEROID BIOCHEM, vol. 25, no. 5a, 1986, pages 677-682, XP000196193 S. STOESSEL ET AL:

## Description

L'invention concerne, à titre de produits industriels nouveaux et utiles, des composés hétérocycliques aromatiques à chaine latérale aromatique. Elle concerne également l'utilisation de ces nouveaux composés dans des compositions pharmaceutiques destinées à un usage en médecine humaine ou vétérinaire, ou bien encore dans des compositions cosmétiques. Elle concerne également des produits intermédiaires susceptibles de donner après réactions les produits de la présente invention.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

Il a été décrit dans J. Agric. Food Chem., vol. 25, no. 4, 1977, p. 723-6, Tetrahedron, vol. 42, no. 3,1986, p. 863-876, Mokuzai Gakkaishi, vol. 33, no. 3, 1987, 234-238, J. Chem. Res., Synop., no. 10, 1985, p. 305, Chem. Abstr., vol. 104, no. 12 1986, abstr. no 95474m des composés de type isoflavonoïdes. Dans la demande brevet EP 0025598, il a été décrit des dérivés 2-phényltétraline et des hétérocycles analogues. Par ailleurs, dans le domaine des rétinoïdes, la demande de brevet EP 0199636 décrit des composés présentant un groupement naphtalénique.

Les composés selon l'invention peuvent être représentés par la formule générale (I) suivante : 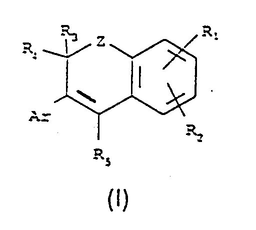 dans laquelle:
* Z représente un radical divalent choisi parmi les radicaux -O-, -S- ou -Nr'-,
* Ar représente soit le radical de formule (II) suivante : 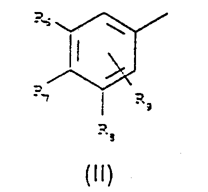 soit le radical de formule (III) suivante: 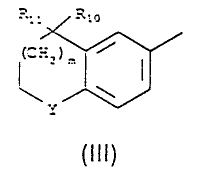 avec m est égal a 0 ou 1
* R₁ représente
   (i) un atome d'hydrogène,
   (ii) le radical -CH₃,
   (iii) le radical -(CH₂)ₚ-O-R₁₂,
   (iv) un radical -OR₁₂,
   (v) un radical 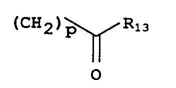
   (vi) un radical -S(O)ₜR₁₄,
      R₁₂, R₁₃, R₁₄, p et t ayant les significations données ci-après,
* R₂ représente un atome d'hydrogène, un atome d'halogène, un radical alkyle inférieur ou le radical -OR₁₂,
   R₁₂ ayant la signfication donnée ci-après,
* R₃ et R₄, identiques ou différents, représentent un atome d'halogène, un atome d'hydrogène, un radical alkyle inférieur ou un radical OR₁₂,
   R₁₂ ayant la signfication donnée ci-après,
* R₅ représente un atome d'halogène, un atome d'hydrogène, un radical alkyle inférieur ou un radical OR₁₅,
   R₁₅ ayant la signfication donnée ci-après,
* R₆, R₇, R₈, R₉, identiques ou différents, sont choisis parmi:
   - un atome d'hydrogène,
   - un atome d'halogène,
   - un radical alkyle,
   - un radical cycloalkyle,
   - un radical -(Z₁)ₙ-(CH₂)_{q}-CO-R₁₃,
   - un radical -Z₂-R₁₂,
   avec au moins deux des radicaux R₆, R₇, R₈ et R₉ différents de l'atome d'hydrogène, et au moins un des radicaux R₆, R₇, R₈ et R₉ représentant un radical alkyle ou un radical cycloalkyle, et R6 différent d'un radical méthyle quand R9 en position para par rapport à R6 est un radical hydroxy,
   Z₁, Z₂, R₁₂, R₁₃, n, q ayant les significations données ci-après,
* R₁₀ et R₁₁ représentent des radicaux alkyles inférieurs,
* Y représente un radical divalent choisi parmi les radicaux : -C(R₁₁)₂-, -O-, -S-, -Nr'-, -CH(OH)-, -CO-, -SO- et -SO₂-,
   R₁₁ étant tel que défini ci-dessus,
   r' ayant la signification donnée ci-après,
   étant entendu que :
   - R₁, R₂, R₃, R₄ et R₅ ne représentent pas simultanément l'atome d'hydrogène,
   - R₁₂ représente un atome d'hydrogène, un radical alkyle inférieur, un radical aryle, un radical aralkyle, un radical polyhydroxyalkyle ou un radical polyether, un radical acyle inférieur,
   - R₁₃ représente :
      (a) un atome d'hydrogène,
      (b) un radical 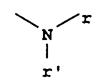 r et r' ayant les significations données ci-après,
      (c) un radical -OR₁₄
         R₁₄ ayant la signification donnée ci-après
   - R₁₄ représente un atome d'hydrogène; un radical alkyle; un radical mono ou polyhydroxyalkyle; un radical aryle ou aralkyle, éventuellement substitué par un ou plusieurs atomes d'halogène, une fonction hydroxyle ou une fonction nitro, ou un groupe méthoxy; ou un reste de sucre ou d'aminoacide,
   - R₁₅ représente un atome d'hydrogène, un radical alkyle inférieur, un radical aryle, un radical aralkyle ou un radical polyether,
   - r et r', identiques ou différents, représentent un atome d'hydrogène; un radical alkyle inférieur; ou un radical aryle, éventuellement substitué par un ou plusieurs atomes d'halogène, une fonction hydroxyle ou une fonction nitro, ou un groupe méthoxy; ou un reste d'amino acide ou de sucre; ou encore, pris ensemble, forment un hétérocycle,
   - Z₁ représente O, S ou Nr',
   - Z₂ représente O ou S,
   - n est égal à 0 ou 1,
   - p est égal à 0, 1, 2 ou 3
   - q est un entier prenant une valeur de 0 à 10,
   - t est égal à 0, 1, 2 ou 3,
   et les isomères optiques et géométriques desdits composés de formule (I), ainsi que leurs sels .

Lorsque les composés selon l'invention se présentent sous forme de sels, par addition d'une base, il s'agit de sels d'un métal alcalin ou alcalino-terreux ou encore de zinc ou d'une amine organique.

Lorsque les composés se présentent sous forme de sels, par addition d'un acide, il s'agit de sels pharmaceutiquement ou cosmétiquement acceptables obtenus par addition d'un acide minéral ou organique, en particulier l'acide chlorhydrique, sulfurique, acétique, citrique, fumarique, hémisuccinique, maléique et mandélique.

De préférence, R₁ et R₂ ne sont pas simultanément soit l'atome d'hydrogène, soit respectivement l'atome d'hydrogène et l'atome d'halogène.

Par radical alkyle inférieur, on entend un radical ayant de 1 à 6 atomes de carbone et de préférence les radicaux méthyle, éthyle, isopropyle, butyle et tertiobutyle.

Par radical acyle inférieur, on entend un radical ayant de 1 à 6 atomes de carbone et, de préférence, les radicaux acétyle, propionyle ou pivaloyle,

Par groupement protecteur de fonction amine, on entend les groupements correspondants décrits dans "Protecting groups in organic synthesis" by T.W Greene, Ed. by John Wiley and Sons (1981).

Par radical cycloalkyle, on entend un radical alcane cyclique ou polycyclique contenant de 1 à 10 atomes de carbone, éventuellement substitué par un ou des atomes d'halogène ou un ou des radicaux hydroxyle et de préférence les radicaux adamantyle ou 1-méthylcyclohéxyle.

Par radical polyéther, on entend un radical ayant de 1 à 6 atomes de carbone et de 1 à 3 atomes d'oxygène ou de soufre tel que les radicaux méthoxyméthyl éther, méthoxyéthoxyméthyl éther ou méthylthyométhyl éther.

Par radical monohydroxyalkyle, on entend un radical ayant de préférence 2 ou 3 atomes de carbone, notamment un radical 2-hydroxyéthyle, 2-hydroxypropyle ou 3-hydroxypropyle.

Par radical polyhydroxyalkyle, on entend un radical contenant de 1 à 6 atomes de carbone et de 1 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Parmi les radicaux aryles, éventuellement substitués, on préfère un radical phényle, éventuellement substitué par un ou plusieurs atomes d'halogène, une fonction hydroxyle ou fonction nitro, ou un groupe méthoxy.

Parmi les radicaux aralkyles, éventuellement substitués, on préfère le radical benzyle ou phénéthyle, éventuellement substitué par un ou plusieurs atomes d'halogène, une fonction hydroxyle ou nitro, ou un groupe méthoxy.

Par reste d'aminoacide, on entend un reste dérivant notamment de l'un des 20 aminoacides de configuration L ou D constitutifs des protéines de mammifères. On choisit plus particulièrement les restes dérivant de la lysine, de la glycine, ou de l'acide aspartique.

Par reste de sucre, on entend un reste dérivant notamment du glucose, du galactose, du mannose ou de l'acide glucuronique.

Parmi les hétérocycles, on préfère un radical pipéridino, morpholino, pyrrolidino ou pipérazino, éventuellement substitué en position 4 par un radical alkyle en C₁-C₆ ou mono ou polyhydroxyalkyle, tels que définis ci-dessus.

Parmi les composés de formule (I) entrant dans le cadre de la présente invention, on peut notamment citer les exemples suivants:
- Acide 3-[(3-(1- adamantyl)-4-méthoxyphényl)-2H-1-benzopyran-7-yl] carboxylique
- 3-[(3-(1-adamanthyl)-4-méthoxyphényl)-2H-1-benzopyran-7-yl] carboxylate de méthyle
- 3-[(3-(1-adamanthyl)-4-hydroxyphényl)-2H-1-benzopyran-7-yl] carboxylate de méthyle
- Acide 3-[(3-(1- adamantyl)-4-hydroxyphényl)-2H-1-benzopyran-7-yl] carboxylique
- Acide 3-[(3- (1-adamantyl)-4-méthoxyphényl)-2H-1-benzopyran-6-yl] carboxylique
- 3-[(3-(1-adamantyl)-4-méthoxyphényl)-2H-1-benzopyran-6-yl] carboxylate de méthyle
- Acide 3-[(3-(1- adamantyl)-4-hydroxyphényl)-2H-1-benzopyran-6-yl] carboxylique
- Acide 3-[(5,6,7,8-tétrahydro-5,5,8,8-tétraméthyl-2-naphthyl) -2H-1-benzopyran-7-yl] carboxylique
- 3-[(5,6,7,8-tétrahydro-5,5,8,8-tétraméthyl-2-naphtyl) -2H-1-benzopyran-7-yl] carboxylate ce méthyle
- Acide 3-[(5,6,7,8-tétrahydro-5,5,8,8-tétraméthyl-2-naphtyl) -2H-1-benzopyran-6-yl] carboxylique
- 3-[(5,6,7,8-tétrahydro-5,5,8,8-tétraméthyl-2-naphtyl) -2H-1-benzopyran-6-yl] carboxylate de méthyle
- 3-[(3,5-Di-tert-butyl-4-méthoxyphényl) -2H-1-benzopyran-6-yl] carboxylate de méthyle
- Acide 3-[(3,5-Di-tert-butyl-4-méthoxyphényl) -2H-1-benzopyran-6-yl] carboxylique
- 3-[(3,5-Di-tert-butyl-4-méthoxyphényl) -2H-1-benzopyran-7-yl] carboxylate de méthyle
- Acide 3-[(3,5-Di-tert-butyl-4-hydroxyphényl) -2H-1-benzopyran-7-yl] carboxylique
- 3-[(4-(1-adamantyl)-3-méthoxyphényl)-2H-1benzopyran]-6-carboxylate de méthyle
- acide 3-[(4-(1-adamantyl)-3-méthoxyphényl)-2H-1benzopyran]-6-carboxylique
- 3-[(3-tert-butyl-4-hydroxy phenyl)-2H-1-benzopyran]-7-carboxylate de méthyle
- Acide-3-[(3-tert-butyl-4-hydroxy phenyl)-2H-1-benzopyran]-7- carboxylique
- 3-[(3-(1-adamantyl)-4-méthoxyethoxyméthoxyphényl)-2H-1-benzopyran]-7-carboxylate de méthyl
- Acide 3-[(3-(1-adamantyl)-4-méthoxyethoxyméthoxyphényl)-2H-1-benzopyran]-7-carboxylique
- 3-[(5,6,7,8-tétrahydro-5,5,8,8-tétraméthyl-2-naphtyl)-2H-1-benzopyran]-7-méthyl alcohol
- 3-[(5,6,7,8-tétrahydro-5,5,8,8-tétraméthyl-2-naphtyl)-2H-1-benzopyran]-7-carboxaldéhyde
- 3-[(3,5-Di-tert-butyl-4-hydroxy phenyl)-2H-1-benzopyran]-7-carboxanilide
- Morpholide de l'acide 3-[(5,6,7,8-tétraydro-5,5,8,8-tétraméthylnaphtyl)-2H-1-benzopyran]-7-carboxylique
- Morpholide de l'acide 3-[(5,6,7,8-tétrahydro-5,5,8,8-tétraméthylnaphtyl)-2H-1-benzopyran]-7-glyoxylique
- N-butyl-3-[(5,6,7,8-tétrahydro-5,5,8,8-tétraméthylnaphtyl)-2H-1-benzopyran]-7-carboxamide.

Selon la présente invention, les composés de formule (I) plus particulièrement préférés sont ceux pour lesquels l'une au moins, et de préférence toutes, les conditions ci-dessous sont respectées:
- R1 est un radical -(CH₂)ₚ-CO-O-R₁₄
- R₂, R₃, R₄, R₅, R₆ et R₉ sont des atomes d'hydrogène
- R7 est un radical cycloalkyle
- R₈ est un radical -OR₁₂
- Z est un atome d'oxygène
- Y est un radical C(R₁₁)₂.

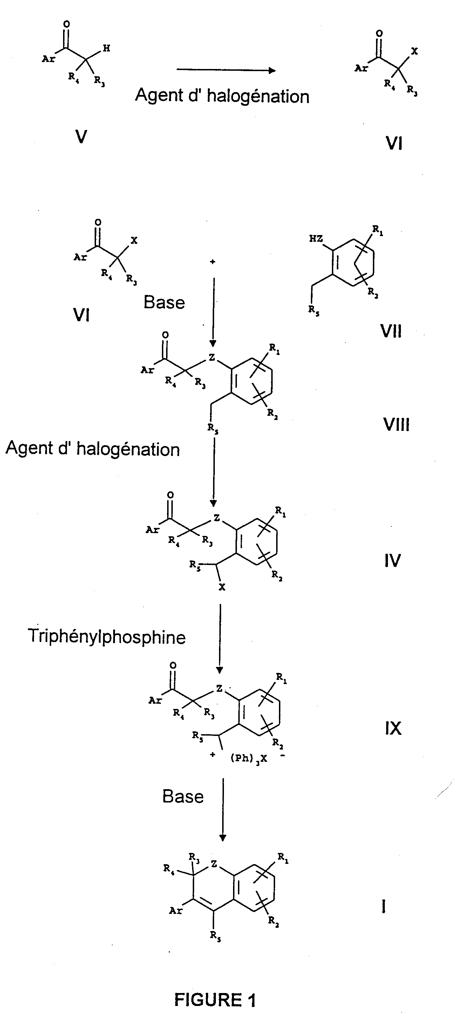

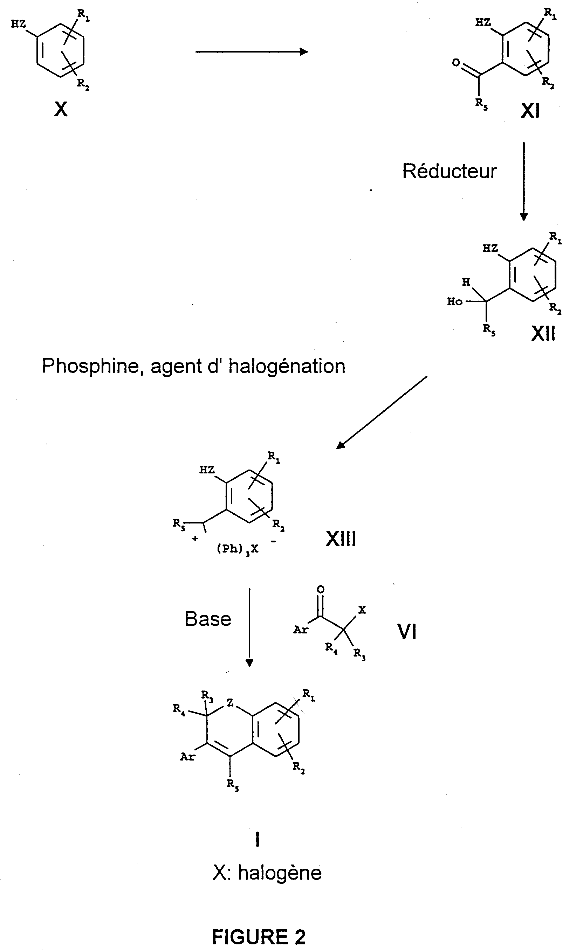

La présente invention a également pour objet les procédés de préparation des composés de formule (I), en particulier selon les schémas réactionnels donnés aux figures 1 et 2.

Les composés intermédiaires pour la synthèse des composés de formule générale (I) peuvent ainsi répondre à la formule (IV) suivante: 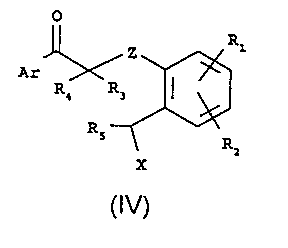
R₁, R₂, R₃, R₄, R₅, Z et Ar étant tels que définis ci-dessus,
X représente un atome d'hydrogène, un atome d'halogène ou un radical hydroxy.

Parmi les composés de formule générale (IV), on peut notamment citer les exemples suivants:
- 4-[(5,6,7,8-tétrahydro-5,5,8,8-tétraméthylnaphtoyl) méthyloxy]-3-méthyl benzoate de méthyle
- 4-[5,6,7,8-tétrahydro-5,5,8,8-tétraméthylnaphtoyl) méthyloxy]-3-bromométhylbenzoate de méthyle
- 3-[(3-(1-adamanthyl)-4-méthoxybenzoyl) méthyloxy]4-méthylbenzoate de méthyle
- 3-[(3-(1-adamanthyl)-4-méthoxybenzoyl) méthyloxy]-4-bromométhylbenzoate de méthyle
- 3-[(5,6,7,8-tétrahydro-5,5,8,8-tétraméthylnaphtoyl) méthyloxy]-4-méthyl benzoate de méthyle
- 3-[(5,6,7,8-tétrahydro-5,5,8,8-tétraméthylnaphtoyl) méthyloxy]-4-bromométhylbenzoate de méthyle
- 3-[(3,5-Di-tert-butyl-4-méthoxybenzoyl) méthyloxy]-4-méthylbenzoate de méthyle
- 3-[(3,5-Di-tert-butyl-4-hydroxybenzoyl)méthyloxy]-4-méthylbenzoate de méthyle.

Selon la présente invention, les composés de formule (IV) plus particulièrement préférés sont ceux pour lesquels l'une au moins, et de préférence toutes, les conditions ci-dessous sont respectées :
- R1 est un radical -(CH₂)ₚ-CO-O-R₁₄
- R₂, R₃, R₄, R₅, R₆ et R₉ sont des atomes d'hydrogène
- R7 est un radical cycloalkyle
- R₈ est un radical -OR₁₂
- Z est un atome d'oxygène
- Y est un radical C(R₁₁)₂
- X est un atome d'hydrogène ou un atome d'halogène.

Ainsi les composés de formule générale (I) peuvent être obtenus (figure 1) à partir de la cétone (V), par halogénation, par exemple au moyen d'un agent de bromation tel que le brome. Le composé (VI) obtenu est ensuite couplé avec le composé (VII), en présence de base telle que le carbonate de potassium ou l'hydrure de sodium. Le dérivé couplé (VIII) est halogéné en position benzylique par action d'un agent d'halogénation tel qu'un N-bromosuccinimide ou un N-chlorosuccinimide. L'halogénure (IV), est converti en ylure de phosphore par action d'une phosphine telle que la triphénylphosphine. Le sel de phosphonium (IX) est cyclisé par addition d'une base comme le méthanolate de sodium pour donner (I).

Les composés de formule générale (I) peuvent aussi être obtenus après couplage et cyclisation entre l'intermédiaire (VI) et un sel de phosphonium (XIII) (figure 2). Dans ce cas, le dérivé (XIII) est obtenu selon la séquence suivante. Par exemple, par formylation aromatique, au moyen de l'hexaméthylènetétramine dans l'acide trifluoroacétique du composé (X), on obtient le composé (XI). Le produit carbonylé (XI) est ensuite réduit par action d'un réducteur tel que le borhydrure de sodium, pour conduire au composé (XII). Le sel de phosphonium (XIII) est obtenu par action de phosphine en présence d'agent d'halogénation tel que CBr₄ ou l'acide bromhydrique. La condensation et la cyclisation se font en présence d'une base.

Les produits de formule générale (I) peuvent servir de produits de départ pour la fabrication d'autres composés. Ces dérivés seront obtenus selon les méthodes classiques de synthèse employées en chimie, telles que celles décrites dans "Advanced Organic Chemistry" de J. March; Jonh Wiley and Sons, 1985.

Par exemple, on peut procéder aux modifications fonctionnelles du groupe R₁ comme indiqué ci-dessous:
- acide carboxylique: -> ester
- ester: -> acide carboxylique
- acide: -> chlorure d' acide
- chlorure d' acide: - > amide
- acide: -> amide
- acide: -> alcool
- alcool: -> aldéhyde
- amide: ->amine
- thiol: ->thioéther
- thioéther: -> sulfoxyde
- thioéther: -> sulfone
- acide sulfonique: -> ester sulfonique
- acide sulfonique: -> sulfonamide
- acide sulfinique: -> ester sulfinique

Les composés de formule générale (I) présentent une activité agoniste ou antagoniste vis-à-vis de l'expression d'un ou plusieurs marqueurs biologiques dans le test de différenciation des cellules (F9) de tératocarcinome embryonnaire de la souris (Skin Pharmacol. 3, p.256-267, 1990) et/ou sur la différenciation des kératinocytes humains in vitro (Skin Pharmacol. 3 p.70-85, 1990) en réponse à un traitement par des rétinoïdes. Ces tests sus-mentionnés montrent les activités des composés dans les domaines de la différenciation et de la prolifération. Leurs activités peuvent aussi être mesurées dans des tests de transactivation cellulaire à l'aide de récepteurs recombinants RARs ou RXRs préalablement transfectés. (B.A. Bernard et al., Biochemical and Biophysical Research Communication 1992, vol. 186, 977-983; M.F. Boehm et al. Journal of Medicinal Chemistry, 1994, 37, 408-414).

La présente invention a également pour objet à titre de médicament les composés de formule (I) tels que décrits ci-dessus.

Les composés selon l'invention conviennent particulièrement bien dans les domaines de traitement suivants :
1) Pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle.
2) Pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen cutané ou muqueux (buccal).
3) Pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; les composés peuvent également être utilisés dans certaines affections inflammatoires ne présentant pas de trouble de la kératinisation.
4) Pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires.
5) Pour traiter d'autres désordres dermatologiques tels que les dermatoses bulleuses et les maladies du collagène.
6) Pour traiter certains troubles ophtalmologiques, notamment les cornéopathies.
7) Pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique.
8) Pour prévenir ou guérir les stigmates de l'atrophie épidermique et/ou dermique induite par les corticostéroïdes locaux ou systémiques, ou tout autre forme d'atrophie cutanée.
9) Pour prévenir ou traiter les troubles de la cicatrisation ou pour prévenir ou réparer les vergetures.
10) Pour lutter contre les troubles de la fonction sébacée tels que l'hyperséborrhée de l'acné ou la séborrhée simple.
11) Dans le traitement ou la prévention des états cancéreux ou précancéreux.
12) Dans le traitement d'affections inflammatoires telles que l'arthrite.
13) Dans le traitement de toute affection d'origine virale au niveau cutané ou général.
14) Dans la prévention ou le traitement de l'alopécie.
15) Dans le traitement d'affections dermatologiques ou générales à composante immunologique.
16) Dans le traitement d'affections du système cardiovasculaire telles que l'artériosclérose.

Dans les domaines thérapeutiques mentionnés ci-dessus, les composés selon l'invention peuvent être avantageusement employés en combinaison avec d'autres rétinoïdes, avec la vitamine D ou ses dérivés, avec des corticostéroïdes ou des estrogènes, en association avec des anti-oxydants, avec des α-hydroxy ou α-céto acides ou leurs dérivés, ou encore avec des bloqueurs de canaux potassiques.

Parmi les autres rétinoïdes, on peut citer notamment l'acide rétinoïque tout-trans, l'acide rétinoïque 9-cis, ou encore un analogue synthétique se liant sur des récepteurs de type RXR ou RAR.

Par vitamines D ou leurs dérivés, on entend par exemple les dérivés de la vitamine D₂ ou D₃ et en particulier la 1,25-dihydroxyvitamine D₃.

Par antiradicaux libres, on entend par exemple l'α-tocophérol, la Super Oxyde Dismutase, l'Ubiquinol ou certains chélatants de métaux.

Par α-hydroxy ou α-céto acides ou leurs dérivés, on entend par exemple les acides lactique, malique, citrique, glycolique, mandélique, tartrique, glycérique,ascorbique ou dérivés d'acide salicylique ou leurs sels, amides ou esters.

Par bloqueurs de canaux potassiques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I), telle que définie ci-dessus, l'un de leurs isomères optiques ou géométriques ou un de leurs sels.

La présente invention a donc aussi pour objet une nouvelle composition médicamenteuse destinée notamment au traitement des affections susmentionées, caractérisée par le fait qu'elle comporte, dans un support pharmaceutiquement acceptable au moins un composé de formule (I), l'un de leurs isomères optiques ou géométriques ou un de leurs sels.

L'administration des compositions pharmaceutiques selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, les compositions peuvent se présenter sous forme de solutions ou suspensions pour perfusion ou pour injection.

Les composés selon l'invention sont généralement administrés à une dose journalière d'environ 0,01 mg/kg à 100 mg/kg en poids corporel en 1 à 3 prises.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent se présenter soit sous forme anhydre, soit sous forme aqueuse selon l'indication clinique.

Par voie oculaire, ce sont principalement des collyres.

Ces compositions pour la voie topique ou oculaire contiennent au moins un composé de formule (I), tel que défini ci-dessus, l'un de ses isomères optiques ou géométriques ou un de ses sels, à une concentration de préférence comprise entre 0,001 et 5 % par rapport au poids total de la composition.

Les composés de formule (I), selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches, pour prévenir et/ou lutter contre le vieillissement photo-induit ou chronologique.

Dans le domaine cosmétique, les composés selon l'invention peuvent être avantageusement employés en combinaison avec d'autres rétinoïdes, avec les vitamines D ou leurs dérivés, avec des corticostéroïdes, en association avec des anti-radicaux libres, avec des α-hydroxy ou α-céto acides ou leurs dérivés, ou encore avec des bloqueurs de canaux ioniques.

Les différents produits pris en association avec les composés de la présente invention sont tels que définis ci-dessus.

La présente invention vise donc également une composition cosmétique contenant, dans un support cosmétiquement acceptable, au moins un composé de formule (I), l'un de ses isomères optiques ou géométriques ou l'un de ses sels, cette composition se présentant notamment sous forme d'une crème, d'un lait, d'une lotion, d'un gel, de microsphères ou nanosphères ou vésicules lipidiques ou polymériques, d'un savon ou d'un shampooing.

La concentration en composés de formule (I) dans les compositions cosmétiques est comprise entre 0,001 et 3 % en poids.

Les compositions médicamenteuses et cosmétiques selon l'invention peuvent, en outre, contenir des additifs inertes ou même pharmacodynamiquement ou cosmétiquement actifs ou des combinaisons de ces additifs et notamment : des agents mouillants; des agents dépigmentants tels que l'hydroquinone, l'acide azelaïque, l'acide caféïque ou l'acide kojique; des émollients; des agents hydratants comme le glycérol, le PEG 400, la thiamorpholinone et ses dérivés ou l'urée; des agents antiséborrhéiques ou antiacnéiques, tels que la S-carboxyméthylcystéine, la S-benzyl-cystéamine, leurs sels et leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4- benzothiadiazine 1,1-dioxyde) et le Phénytoïn (5,4-diphénylimidazolidine 2,4-dione) ; des agents anti-inflammatoires non stéroïdiens; des caroténoïdes et, notamment le β-carotène; des agents anti-psoriatiques tels que l'anthraline et ses dérivés et enfin, les acides eicosa-5,8,11,14- tétraynoïque et eicosa-5,8,11-triynoïque, leurs esters et les amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration et sans aucun caractère limitatif, plusieurs exemples de préparation des composés actifs selon l'invention ainsi que des exemples de compositions les contenant.

Dans tout ce qui suit ou ce qui précède, les pourcentages sont exprimés en poids, sauf mention contraire.

### A. EXEMPLES DE COMPOSES

### 1) SELON LA VOIE DE SYNTHESE ILLUSTREE PAR LA FIGURE 1

### EXEMPLE 1

### Acide 3-[(3-(1-adamantyl)-4-méthoxyphényl)-2H-1-benzopyran-7-yl] carboxylique.

3-Adamantyl 4- méthoxy acétophénone.
1/ On chauffe à 50°C de l' acide 3-adamantyl 4-méthoxy benzoïque (50 g, 0,17 mol) dans le toluène. On additionne alors, le chlorure de thionyle (25 ml, 0,34 mol). Après deux heures de chauffage à reflux, le mélange est concentré à sec.
2/ On ajoute goutte à goutte du tétraméthyl étain (6ml), à une solution de chlorure de 3-adamantyl 4-méthoxy benzoyle (12 g, 39 mmol) dans l'héxaméthyl phosphore triamide (45 ml). Le BnPd(PPh3)₂Cl (30 mg, 398 mmol) est ensuite introduit. Le mélange est chauffé à 65°C pendant une heure, puis laissé sous agitation pendant deux jours à température ambiante. Après extraction à l' éther, lavage à l'eau et séchage sur sulfate de sodium, la phase organique est concentrée à l'évaporateur rotatif sous vide. Le produit est purifié par chromatographie flash (CH₂Cl₂ 65 %, hexane 35 %). Solide blanc. Masse: 6,67 g. Rendement: 60%.
   RMN ¹H (CDCL₃, 250 MHz): 1,77 (6H, s), 2,10 (9H, s), 2,56 (3 H, s), 3,90 (3H, s), 6,88 (1H Ar, d), 7,82 (1H Ar, m), 7,88 (1H Ar, d).

3-Adamantyl 4-méthoxy bromoacétophénone.

La 3-adamantyl 4-méthoxy acétophénone (11,41 g,, 40 mmol) est dissoute dans 60 ml de dioxane et 60 ml d' éther éthylique. On ajoute goutte à goutte le brome (2,3ml, 45 mmol), dilué dans 20 ml de dichlorométhane. On agite une heure à température ambiante. On verse le milieu réactionnel, dans 100 ml d' un mélange eau et glace. Après agitation et décantation, la phase organique est lavée deux fois par 100 ml d' eau, séchée sur sulfate de magnésium et concentrée à l' évaporateur rotatif sous vide à 40 °C. Le produit est purifié par chromatographie flash sur colonne de silice (CH₂Cl₂). Solide blanc. Masse: 14,52 g. Rendementt: quantitatif
RMN ¹H (CDCL₃, 250 MHz): 1,78 (6H, s), 2,09 (9H, s), 3,92 (3 H, s), 4,41 (2H, s), 6,91 (1H Ar, d), 7,86 (1H Ar, m), 7,91 (1H Ar, d).

3-[(3-(1-adamanthyl)-4-méthoxybenzoyl) méthyloxy]-4-méthyl benzoate de méthyle.

Une solution de 3-adamantyl 4-méthoxy bromoacétophénone (1g, 2,74 mmol), de 3-hydroxy 4-méthyl benzoate de méthyle (0,46 g, 2,74 mmol),et de carbonate de potassium (0,38 g, 2,74 mmol) dans la méthyl éthyl cétone (20 ml), est chauffée à reflux pendant trois heures. On filtre le milieu réactionnel, puis on ajoute 40 ml d'eau et 40 ml d' éther éthylique. Après agitation et décantation, la phase organique est lavée deux fois par 40 ml d' eau, séchée sur sulfate de magnésium et concentrée à l' évaporateur rotatif sous vide à 40 °C. Le produit est purifié par chromatographie flash sur colonne de silice (acétate d' éthyle 30 %, heptane 70 %).
Solide blanc. Masse: 1,07 g, Rendement: 87%. Pf: 143°C
RMN ¹H (CDCL₃, 250 MHz): 1,77 (6H, s), 2,09 (9H, s), 2,47 (3H, s), 3,88 (3H, s), 3,92 (3H, s), 5,31 (2H, s), 6,92 (1H Ar, d), 7,22 (1H Ar, d), 7,43 (1H Ar, s), 7,59 (1H Ar, s), 7,88 (2H, m).

3-[(3-(1-adamanthyl)-4-méthoxy benzoyl) méthyloxy]-4-bromo méthyl benzoate de méthyle.

Une solution de 3-[(3-(1-adamanthyl)-4-méthoxy benzoyl) méhtyloxy]-4-méthyl benzoate de méthyl. (2,36 g, 5,26 mmol), de péroxyde de benzoyle (0,02 g), dans le tétrachlorure de carbone (50 ml) est chauffée à reflux. On rajoute du péroxyde de benzoyle (0,03 g) et de la N-bromo succinimide ( 1,05 g, 5,9 mmol). Le mélange est chauffé quatre heures à reflux. On additionne 50 ml d' eau et 50 ml de dichlorométhane. Après décantation, la phase organique est lavée deux fois, par 50 ml d' eau, et séchée sur sulfate de magnesium. Après concentration à l' évaporateur rotatif à 40°C, sous vide, le produit désiré est isolé par chromatographie flash (CH₂Cl₂).
Solide blanc. Masse: 1,8g. Rendement: 34%. Pf: 165°C
RMN ¹H (CDCL₃, 250 MHz): 1,78 (6H, s), 2,10 (9H, s), 3,89 (3H, s), 3,93 (3H, s), 4,66 (2H, s), 5,42 (2H, s), 6,93 (1H Ar, d), 7,44 (2H Ar, m), 7,65 (1H Ar, d), 7,89 (2H, m).

3-[(3-(1-adamanthyl)-4-méthoxy phényl)-2H-1-benzopyran-7-yl] carboxylate de méthyle.

On chauffe à reflux un mélange de 3-[(3-(1-adamanthyl)-4-méthoxy benzoyl) méhtyloxy]-4-bromo méthyl benzoate de méthyle (0,53 g, 1 mmol), de triphénylphosphine (0,29 g, 1,1 mmol), dans le tétrahydrofurane (5 ml), quatre heures, sous argon. On refroidit à température ambiante, et on ajoute goutte à goutte une solution de méthylate de sodium à 30% dans le méthanol (0,18 g, 1 mmol). On agite trente minutes à température ambiante. On évapore le solvant à I' évaporateur rotatif sous vide. On additionne 20 ml d' eau et 80 ml dichlorométhane. La phase organique est lavée deux fois par 20 ml d' une solution saturée de chlorure de sodium, et séchée sur sulfate de magnésium. Après concentration à l' évaporateur rotatif à 40°C sous vide, le solide obtenu est lavé à l' éthanol.
Solide blanc. Masse: 0,3 g, Rendement: 70 %.Pf: 209°C
RMN ¹H (CDCL₃, 250 MHz): 1,79 (6H, s), 2,12 (9H, s), 3,87 (3H, s), 3,90 (3H, s), 5,19 (2H, s), 6,73 (1H, s), 6,89 (1H Ar, d), 7,11 (1H Ar, d), 7,24 (1H Ar, d), 7,36 (1H Ar, s), 7,48 (1H Ar, s), 7,59 (1H Ar, d).

Acide 3-[(3-(1-adamanthyl)-4-méthoxyphényl)-2H-1-benzopyran-7-yl] carboxylique.

Une solution de 3-[(3-(1-adamanthyl)-4-méthoxyphényl)-2H-1-benzopyran-7-yl] carboxylate de méthyle (0,12 g, 0,28 mmol), de soude (11 mg, 1,5 mmol), dans le méthanol (1,5 ml) est chauffée douze heures à reflux. Le méthanol est éliminé par distillation à l' évaporateur rotatif. On rajoute 3 ml d' eau et on acidifie à ph=1, par une solution d'acide chlorhydrique à 32 %. Après filtration, le solide obtenu est lavé à l' hexane et séché sous vide à 40°C.
Solide jaune pâle. Masse: 0,1g, Rendement: 86%
RMN ¹H (DMSO, 250 MHz): 1,75 (6H, s), 2,09 (9H, s), 2,47 (3H, s), 3,84 (3H, s), 5,21 (2H, s), 6,61(1H, s), 6,87 (1H Ar, s), 7,01 (1H Ar, d), 7,27 (1H Ar, d), 7,38 (2H Ar, m), 7,49 (H, d).

### EXEMPLE 2

### Acide 3-[(5,6,7,8-tétrahydro-5,5,8,8-tétraméthylnaphtyl)-2H-1-benzopyran-7-yl] carboxylique.

3-[(5,6,7,8-tétrahydro-5,5,8,8-tétraméthylnaphtoyl) méthyloxy]-4-méthyl benzoate de méthyle.

Une solution de 5,6,7,8-tétrahydro-5,5,8,8-tétraméthyl bromoacétonaphtone (1g, 2,74 mmol), de 3-hydroxy 4-méthylbenzoate de méthyle (0,46 g, 2,74 mmol),et de carbonate de potassium (0,38 g, 2,74 mmol) dans la méthyl éthyl cétone (20 ml), est chauffée à reflux pendant trois heures. On filtre le milieu réactionnel, puis on ajoute 40 ml d'eau et 40 ml d' éther éthylique. Après agitation et décantation, la phase organique est lavée deux fois par 40 ml d' eau, séchée sur sulfate de magnésium et concentrée à l' évaporateur rotatif sous vide à 40 °C. Le produit est purifié par chromatographie flash sur colonne de silice (acétate d' éthyle 30 %, heptane 70 %).
Solide blanc. Masse: 1,07 g, Rendement: 87%. Pf: 99°C
RMN ¹H (CDCL₃, 250 MHz): 1,31 (6H, s), 1,32 (6H, s) , 1,71 (4H, s), 2,35 (3H, s), 3,87 (3H, s), 5,25 (2H, s), 7,18 (1H Ar, d), 7,36 (2H Ar, m), 7,54 (1H Ar, q), 7,69 (1H Ar, q), 7,92 (1H Ar, s).

3-[(5,6,7,8-tétrahydro-5,5,8,8-tétraméthylnaphtoyl) méthyloxy]-4-bromométhyl benzoate de méthyle.

Une solution de 3-[(5,6,7,8-tétrahydro-5,5,8,8-tétraméthylnaphtoyl) méthyloxy]-4-méthyl benzoate de méthyle (7,02 g, 16,7 mmol), de péroxyde de benzoyle (0,02 g) et de N-bromosuccinimide ( 4,28 g, 24 mmol), dans le tétrachlorure de carbone (370 ml) est chauffée à reflux, et irradiée par une lampe 1000 W pendant quarante cinq minutes. On additionne 350 ml d' eau et 250 ml de dichlorométhane. Après décantation, la phase organique est lavée deux fois, par 350 ml d' eau, et séchée sur sulfate de magnesium. Après concentration à l' évaporateur rotatif à 40°C, sous vide, les trois produits obtenus sont purifiés par chromatographie flash (CH₂Cl₂), puis par HPLC (CH₂Cl₂ 70%, heptane 30%). Monobromé benzylique:
Solide blanc. Masse: 1,02g. Rendement: 12,3%, Pf: 142°C.
RMN ¹H (CDCL₃, 250 MHz): 1,20 (12H, s), 1,66 (4H, s), 3,82 (3H, s), 4,61 (2H, s), 5,40 (2H, s), 7,37 (2H Ar, m), 7,59 (1H Ar, q), 7,67 (1H Ar, q), 7,90 (1H Ar, s).

3-[(5,6,7,8-tétrahydro-5,5,8,8-tétraméthylnaphtyl)-2H-1-benzopyran-7-yl] carboxylate de méthyle.

On chauffe à reflux un mélange de 3-[(5,6,7,8-tétrahydro-5,5,8,8-tétraméthyl naphtoyl) méthyloxy]-4-bromométhylbenzoate de méthyle (1 g, 2,01 mmol), de triphénylphosphine (0,59 g, 2,21 mmol), dans le tétrahydrofurane (12 ml), quatre heures, sous argon. On refroidit à température ambiante, et on ajoute goutte à goutte une solution de méthylate de sodium à 30% dans le méthanol (0,37 g, 2,05 mmol). On agite trente minutes à température ambiante. On évapore le solvant à l' évaporateur rotatif sous vide. On additionne 20 ml d' eau et 80 ml d' acétate d' éthyle. La phase organique est lavée deux fois par 20 ml d' une solution saturée de chlorure de sodium, et séchée sur sulfate de magnésium. Après concentration à l' évaporateur rotatif à 40°C sous vide, le solide obtenu est lavé à l' heptane
Solide blanc. Masse: 0,6 g, Rendement: 79 %, Pf: 154°C.
RMN ¹H (CDCL₃, 250 MHz): 1,29 (6H, s), 1,32 (6H, s), 1,70 (4H, s), 3,90 (3H, s), 5,20 (2H, s), 6,77 (1H, s), 7,12 (1H Ar, d), 7,22 (1H Ar, q), 7,33 (1H Ar, s), 7,37 (1H Ar, s), 7,49 (1H Ar, s), 7,59 (1H Ar, s).

Acide 3-[(5,6,7,8-tétrahydro-5,5,8,8-tétraméthylnaphtyl)-2H-1-benzopyran-7-yl] carboxylique.

Une solution de 3-[(5,6,7,8-tétrahydro-5,5,8,8-tétraméthylnaphtyl)-2H-1-benzopyran-7-yl] carboxylate de méthyle (0,6 g, 1,6 mmol), de soude (0,38 g, 9,5 mmol),de méthanol (1ml) et d'eau (1 ml), dans le THF (9 ml) est agitée vingt quatre heures. Le milieu réactionnel est concentré à l'évaporateur rotatif. On rajoute 10 ml d' eau et on acidifie à ph=1, par une solution d'acide chlorhydrique à 32 %. Après filtration, le solide obtenu est solubilisé dans un mélange de 25 ml d' acétate d' éthyle et 10 ml d' heptane. L' acétate d' éthyle est distillé à l' évaporateur rotatif sous vide, à 40°C. On recueille le produit par filtration.
Solide blanc. Masse: 0,489g, Rendement: 84%. Pf: 285°C
RMN ¹H (DMSO, 250 MHz): 1,15 (6H, s), 1,2 (6H, s), 1,56 (4H, s), 5,15 (2H, s), 6,99 (1H, s), 7,24 (4H Ar, m), 7,41 (2H Ar, m), 12,78 (1H, s) .

### EXEMPLE 3

### Acide 3-[(5,6,7,8-tétrahydro-5,5,8,8-tétraméthylnaphtyl)-2H-1-benzopyran-6-yl] carboxylique.

4-[(5,6,7,8-tétrahydro-5,5,8,8-tétraméthylnaphtoyl) méthyloxy]-3-méthylbenzoate de méthyle.

Une solution de 5,6,7,8-tétrahydro-5,5,8,8-tétraméthyl bromoacétonaphtone (5g, 16,2 mmol), de 4-hydroxy 3-méthylbenzoate de méthyle (2,68 g, 16,1 mmol),et de carbonate de potassium (2,23 g, 16,1 mmol) dans la méthyl éthyl cétone (120 ml), est chauffée à reflux pendant trois heures. On filtre le milieu réactionnel, puis on ajoute 250 ml d'eau et 250 ml d' éther éthylique. Après agitation et décantation, la phase organique est lavée deux fois par 40 ml d' eau, séchée sur sulfate de magnésium et concentrée à l' évaporateur rotatif sous vide à 40 °C. Le produit est purifié par chromatographie flash sur colonne de silice (acétate d' éthyle 30 %, heptane 70 %).
Solide blanc. Masse: 5,35 g, Rendement: 84%. Pf: 111°C
RMN ¹H (CDCL₃, 250 MHz): 1,31 (12H, s), 1,71 (4H, s), 2,34 (3H, s), 3,87 (3H, s), 5,34 (2H, s), 6,71 (1H Ar, d), 7,42 (1H Ar, d), 7,73 (1H Ar, q), 7,82 (1H Ar, q), 7,86 (1H Ar, d), 7,98 (1H Ar, d).

4-[(5,6,7,8-tétrahydro-5,5,8,8-tétraméthylnaphtoyl) méthyloxy]-3-bromométhyl benzoate de méthyle.

Une solution de 4-[(5,6,7,8-tétrahydro-5,5,8,8-tétraméthylnaphtoyl) méthyloxy]-3-méthylbenzoate de méthyle.
(3,73 g, 9,5 mmol), de péroxyde de benzoyle (0,02 g) et de N-bromosuccinimide (1,74 g, 9,8mmol), dans le tétrachlorure de carbone (200 ml) est chauffée à reflux, et irradiée par une lampe 1000 W pendant quinze minutes. On additionne 300 ml d' eau et 150 ml de dichlorométhane. Après décantation, la phase organique est lavée deux fois, par 250 ml d' eau, et séchée sur sulfate de magnesium. Après concentration à l' évaporateur rotatif à 40°C, sous vide, le produit obtenu est purifié par chromatographie flash (CH₂Cl₂ 10%, heptane 90%).
Solide blanc. Masse: 0,49 g. Rendement: 10%, Pf: 114°C.
RMN ¹H (CDCL₃, 250 MHz): 1,31 (12H, s), 1,72 (4H, s), 3,88 (3H, s), 4,65 (2H, s), 5,46 (2H, s), 6,76 (2H Ar, d), 7,74 (1H Ar, q), 7,93 (1H Ar, q), 7,96 (1H Ar, d), 8,08 (1H Ar, d).

3-[(5,6,7, 8-tétrahydro-5,5,8,8-tétraméthylnaphtyl)-2H-1-benzopyran-6-yl] carboxylate de méthyle.

On chauffe à reflux un mélange de 4-[(5,6,7,8-tétrahydro-5,5,8,8-tétraméthyl naphtoyl) méthyloxy]-3-bromométhylbenzoate de méthyle (440 mg, 0,88 mmol), de triphénylphosphine (0,26 g, 1 mmol), dans le tétrahydrofurane (5 ml), quatre heures, sous argon. On refroidit à température ambiante, et on ajoute goutte à goutte une solution de méthylate de sodium à 30% dans le méthanol (0,16 g, 0,89 mmol). On agite quarante minutes à température ambiante. On évapore le solvant à l' évaporateur rotatif sous vide. On additionne 40 ml d' eau et 60 ml d' éther éthylique. La phase organique est lavée deux fois par 40 ml d' eau, et séchée sur sulfate de magnésium. Après concentration à l'évaporateur rotatif à 40°C sous vide, le solide obtenu est lavé à l' heptane
Solide blanc. Masse: 0,24 g, Rendement: 72 %, Pf: 144°C.
RMN ¹H (CDCL₃, 250 MHz): 1,29 (6H, s), 1,32 (6H, s), 1,70 (4H, s), 3,89 (3H, s), 5,24 (2H, s), 6,77 (1H, s), 6,84 (1H Ar, d), 7,20 (1H Ar, q), 7,34 (1H Ar, d), 7,78 (1H Ar, d), 7,81 (1H Ar, q).

Acide 3-[(5,6,7,8-tétrahydro-5,5,8,8-tétraméthylnaphtyl)-2H-1-benzopyran-6-yl] carboxylique.

Une solution de 4-[(5,6,7,8-tétrahydro-5,5,8,8-tétraméthylnaphtyl)-2H-1-benzopyran-6-yl] carboxylate de méthyle (0,22 g, 0,58 mmol), de soude (0,1 g, 2,5 mmol), de méthanol (0,5ml) et d' eau (0,5 ml), dans le THF (3 ml) est chauffée quatre heures à reflux. Le milieu réactionnel est concentré à l'évaporateur rotatif. On rajoute 5 ml d' eau et on acidifie à ph=1, par une solution d'acide chlorhydrique à 32 %. Après filtration, le solide obtenu est solubilisé dans un mélange de 10 ml d' acétate d' éthyle, 10 ml de THF et 10 ml d' heptane.Le mélange est concentré à l' évaporateur rotatif sous vide, à 40°C. On recueille le produit par filtration.
Solide blanc. Masse: 0,210g, Rendement: quantitatif. Pf: 271°C
RMN ¹H (DMSO, 250 MHz): 1,26 (6H, s), 1,31 (6H, s), 1,67 (4H, s), 5,29 (2H, s), 6,89 (1H Ar, d), 7,13 (1H, s), 7,31 (1H Ar, d), 7,38 (1H Ar, d), 7,52 (1H Ar, s), 7,73 (1H Ar, d), 7,82 (1H Ar, s).

### 2) SELON LA VOIE DE SYNTHESE ILLUSTREE PAR LA FIGURE 2

### EXEMPLE 4

### 3-[(3-(1-adamantyl)-4-méthoxyphényl)-2H-1benzopyran-6-yl] carboxylate de méthyle

3-formyl-4-hydroxybenzoate de méthyle.

On mélange du 4-hydroxybenzoate de méthyle (4,56 g, 30 mmoles) avec de l'acide trifluoroacétique (24 ml), et de l' hexaméthylènetétramine (8,41 g, 60 mmoles) (réaction de DUFF).
Le milieu réactionnel est chauffé pendant trois heures à 80°C, refroidi à 0°C, puis on ajoute successivement 15 ml d'acide sulfurique à 50%, et 90 ml d'eau déminéralisée.
Après une heure d'agitation à la température ambiante, le milieu est extrait par de l'éther éthylique, lavé à l'eau, séché sur sulfate de magnésium, filtré et les solvants évaporés.
Le produit est purifié par passage sur colonne de silice, éluée par du dichlorométhane.
On obtient, après évaporation des solvants, 1,92 g d'un solide blanc cristallisé, soit un rendement de 36%.
Solide blanc. Masse: 1,92g, Rendement: 36%.
RMN ¹H (CDCL₃, 250 MHz): 3,93 (3H, s), 7,04 (1H Ar, d), 8,19 (1H Ar, Dd), 8,32 (1H Ar, d), 11,40 (1H, s), 9,96 (1H, s),

3-hydroxyméthyll-4-hydroxybenzoate de méthyle.

On mélange du composé obtenu précédemment (1,80 g, 10 mmoles) avec 50 ml de méthanol, et ajoute par petites fractions en maintenant la température inférieure à 20°C, du borohydrure de sodium (189 mg, 5 mmoles) .
Après cinq minutes d'agitation à la température ambiante, on évapore le méthanol, puis le milieu est versé sur un mélange HCI 6N / glace, extrait à l'éther éthylique, lavé à l'eau jusqu'à pH neutre, séché sur sulfate de magnésium, filtré et les solvants évaporés.
Solide blanc. Masse: 1,46g, Rendement: 82%.
RMN ¹H (DMSO, 250 MHz): 3,80 (3H, s), 4,49 (2H, s), 5,14 (1H, s), 6,86 (1H Ar, d), 7,71 (1H Ar, Dd), 7,98 (1H Ar, d), 10,31(1H, s),
Bromure de 2-hydroxy-5-méthoxycarbonylbenzyltriphényl phosphonium.

On mélange successivement du composé obtenu précédemment (182 mg, 1 mmole) avec 2 ml d'acétonitrile et du bromhydrate de triphénylphosphine (361 mg, 1,05 mmole).
Après une heure et quinze minutes de chauffage à reflux, on refroidit le milieu réactionnel, évapore à sec, ajoute de l'éther éthylique, filtre, lave le précipité à l'éther éthylique.
On obtient, après séchage à l'étuve, 470 mg d'un solide blanc, soit un rendement de 93%.
Solide blanc. Masse: 470 mg, Rendement: 93%.
RMN ¹H (DMSO, 250 MHz): 3,70 (3H, s), 5,00 (2H, d), 6,83 (1H Ar, d), 7,40-7,92 (20H Ar, m), 10,79 (1H, s),

### 3-[(3-(1-adamantyl)-4-méthoxyphényl)-2H-1benzopyran-6-yl] carboxylate de méthyle

On additionne goutte à goutte une solution de méthanolate de sodium (0,45 ml, 2,36 mmol), à une solution de bromure de 2-hydroxy-5-méthoxycarbonylbenzyltriphényl phosphonium (1,52 g, 3 mmol) dans le THF (20 ml). On laisse agiter à température ambiante vingt minutes, puis on ajoute du 3-(1-adamantyl)-4-méthoxybromoacétophénone (1,1 g, 3 mmol). On agite à température ambiante trente minutes. On additionne goutte à goutte une solution de méthanolate de sodium (0,84 ml, 4,41 mmol). On chauffe six heures à reflux. Le milieu réactionnel est concentré à l' évaporateur rotatif sous vide à 40°C. On ajoute 20 ml d'eau, puis on acidifie par une solution d'acide chlorhydrique concentré jusqu'à pH=1. On extrait par 30 ml d'acétate d'éthyle. Après décantation la phase organique est lavée deux fois par 20 ml d' eau, séchée sur sulfate de magnésium anhydre et concentré à l' évaporateur rotatif sous vide à 40°C.
Solide blanc. Masse: 418 mg, Rendement: 32%.Pf: 162°C.
RMN ¹H (DMSO, 250 MHz): 1,75 (6H, s), 2,08 (9H, s), 3,82 (6H, s), 5,27 (2H, s), 6,89 (1H Ar, d, J=10 Hz), 7,00 (1H Ar, d, J=10 Hz), 7,07 (1H, s), 7,35 (2H Ar, m), 7,71 (1H Ar, dd, J=1,9 Hz, J=8,5 Hz), 7,83 (1H Ar, d, 1,9 Hz).
RMN ¹³C (DMSO, 250 MHz): 28,50; 36,66; 36,74; 40,10; 51,94; 55,43; 67,09; 112,26; 115,23; 116,37; 122,81; 122,91; 123,04; 123,76; 127,48; 128,11; 130,12; 132,25; 137,93; 156,68; 158,68; 158,96; 165,95.

### EXEMPLE 5

### acide 3-[(3-(1-adamantyl)-4-méthoxyphényl)-2H-1benzopyran-6-yl] carboxylique

Une solution de 3-[(3-(1-adamantyl)-4-méthoxyphényl)-2H-1benzopyran-6-yl] carboxylate de méthyle (0,12 g, 1,28 mmol), de soude (11 mg, 1,5 mmol), dans le méthanol (2 ml) est chauffée douze heures à reflux. Le méthanol est éliminé par distillation à l'évaporateur rotatif sous vide. On rajoute 3 ml d'eau et on acidifie à pH=1, par une solution d' acide chlorhydrique concentré. Après filtration, le solide obtenu est lavé à l'hexane et séchée en étuve.
Solide jaune pâle. Masse: 100 mg, Rendement: 86%.Pf: 259°C.
RMN ¹H (DMSO, 250 MHz): 1,74 (6H, s), 2,07 (9H, s), 3,82 (3H, s), 5,26 (2H, s), 6,89 (1H, d, J=8,4 Hz), 6,98 à 7,05 (2H Ar, m), 7,35 (2H Ar, m), 7,69 (1H Ar, d, J=8,2Hz), 7,81 (1H Ar, s).
RMN ¹³C (DMSO, 250 MHz): 28,41, 36,57, 40,21, 55,35, 66,96, 112,21, 114,98, 116,52, 122,67, 122,95, 123,69, 123,92, 127,53, 128,25, 130,21, 132,06, 137,84, 156,30, 158,83, 166,97.

### EXEMPLE 6

### 3-[(3,5-Di-tert-butyl-4-hydroxy phenyl)-2H-1-benzopyran]-6-carboxylate de méthyle

3,5-di-tert.butyl-4-hydroxy bromoacétophénone.

On ajoute goutte à goutte une solution de 3,5-di-tert-butyl-4-hydroxy acétophénone (2,9 g, 11,5 mmol) dans l'acétate d'éthyle (70 ml), à une suspension de bromure cuivreux (5,2 g, 23 mmol) dans le dichlorométhane à reflux. Le mélange est chauffé 3h, filtré et concentré à l'évaporateur rotatif sous vide à 40°C.
Solide violet. Masse: 2,93 g. Rendement: 78 %; Pf: 104,2°C
RMN ¹H (CDCl₃, 250 MHz): 1,47 (18 H, s), 4,40 (2H, s), 5,92 (1H, s), 7,88 ( H Ar, s).

### 3-[(3,5-Di-tert-butyl-4-hydroxy phenyl)-2H-1-benzopyran]-6-carboxylate de méthyle

On chauffe 1h à reflux une suspension de bromure de 2-hydroxy-5-rnéthoxycarbonylbenzyltriphényl phosphonium (1,52 g, 3 mmol) et de carbonate de potassium (0,41 g, 3 mmol) dans le dioxane (20 ml). La solution est refroidie à température ambiante puis, on additionne goutte à goutte une solution de méthanolate de sodium à 30% dans le méthanol (0,84 ml, 4,41 mmol). On chauffe six heures à reflux. Le milieu réactionnel est concentré à l' évaporateur rotatif sous vide à 40°C. On ajoute 20 ml d'eau, puis on acidifie par une solution d'acide chlorhydrique concentré jusqu'à pH=1. On extrait par 30 ml d'acétate d'éthyle. Après décantation la phase organique est lavée deux fois par 20 ml d' eau, séchée sur sulfate de magnésium anhydre et concentré à l' évaporateur rotatif sous vide à 40°C.
Solide blanc. Masse: 710 mg, Rendement: 60%.Pf: 153°C.
RMN ¹H (CDCl₃, 250 MHz): 1,48 (18H, s), 3,89 (3H, s), 5,23 (2H, s), 5,38 (1H, s), 6,68 (1H, s), 6,84 (1H Ar, d, J=8 Hz), 7,25 (2H Ar, s), 7,78 à 7,82 (2H Ar, m).
RMN ¹³C (CDCl₃, 250 MHz): 30,24, 34,52, 51,92, 67,92, 115,25, 117,25, 121,86, 122,83, 123,34, 127,54, 128,28, 130,47, 133,15, 136,27, 154,40, 157,03, 166,86.

### EXEMPLE 7

### acide-3-[(3,5-Di-tert-butyl-4-hydroxyphenyl)-2H-1-benzopyran]-6-carboxylique.

Une solution de 3-[(3,5-Di-tert-butyl-4-hydroxy phenyl)-2H-1-benzopyran]-6-carboxylate de méthyle (610 mg, 1,55 mmol), de soude (260 mg, 6,2 mmol), D'hydroxyde de lithium (260 mg, 6,2 mmol) ,de méthanol (0,5 ml), d'eau (0,5 ml) dans le THF(5ml) est agitée 4 j à température ambiante. La solution est concentrée à l'évaporateur rotatif sous vide à 40°C. On ajoute 10 ml d'eau, 10 ml d'acétate d'éthyle puis on acidifie à pH=1, par une solution d' acide chlorhydrique concentré. La phase organique est lavée deux fois à l'eau, séchée sur sulfate de magnésium anhydre et concentrée à l'évaporateur rotatif sous vide à 40°C. Le produit est purifié par chromatographie flash sur colonne de silice (acétate d' éthyle 10 %, CH₂Cl₂ 90 %).
Solide blanc. Masse: 50 mg, Rendement: 9%.Pf: 244°C.
RMN ¹H (CDCl₃, 250 MHz): 1,47 (18H, s), 5,33 (2H, s), 5,86 (1H, s), 6,69 (1H, s), 6,81 (1H Ar, d, J=9 Hz), 7,46 (2H Ar, s), 7,79 à 7,82 (2H Ar, m).
RMN ¹³C (CDCl₃, 250 MHz): 14,57, 23,10, 30,66, 34,94, 68,42, 115,82, 122,28, 122,81, 123,045, 123,31, 127,87, 129,34, 131,69, 133,61, 136,71, 154,86, 158,23, 172,37.

### EXEMPLE 8

### 3-[(4-(1-adamantyl)-3-méthoxyphényl)-2H-1benzopyran]-6-carboxylate de méthyle

On chauffe à reflux un mélange de bromure de 2-hydroxy-5-méthoxycarbonylbenzyltriphényl phosphonium (1,4 g, 2,8 mmol), de 4-(1-adamantyl)-3-méthoxybromoacétophénone (0,9 g, 2,5 mmol), de carbonate de potassium (0,35 g, 2,5 mmol) dans le dioxane pendant 2 h. On refroidit à température ambiante puis, on additionne goutte à goutte, une solution de méthanolate de sodium à 30 % dans le méthanol (0,7 ml, 3,7 mmol). On chauffe six heures à reflux. Le milieu réactionnel est concentré à l' évaporateur rotatif sous vide à 40°C. On ajoute 40 ml d'eau et 40 ml d'acétate d'éthyle. On acidifie le mélange par une solution d'acide chlorhydrique concentré jusqu'à pH=1. Après décantation la phase organique est lavée deux fois par 40 ml d' eau, séchée sur sulfate de magnésium anhydre et concentré à l' évaporateur rotatif sous vide à 40°C.
Solide blanc. Masse: 337 mg, Rendement: 32%.Pf: 126°C.
RMN ¹H (CDCl₃, 250 MHz): 1,77 (6H, s), 2,09 (9H, s), 3,89 (3H, s), 5,24 (2H, s), 6,79 (1H , s), 6,84 (1H Ar, d, J=8,5 Hz), 6,93 à 6,96 (2H, m), 7,24 (1H Ar, d, J=9 Hz), 7,79 à 7,84 (2H, m).
RMN ¹³C (CDCl₃, 250 MHz): 29,20, 37,24, 40,68, 52,08, 55,20, 67,84, 108,13, 115,48, 117,19, 118,81, 122,58, 123,54, 127,05, 128,72, 131,04, 132,21, 134,90, 139,55, 157,38, 159,25, 166,89.

### EXEMPLE 9

### acide 3-[(4-(1-adamantyl)-3-méthoxyphényl)-2H-1benzopyran]-6-carboxylique

Une solution de 3-[(4-(1-adamantyl)-3-méthoxyphényl)-2H-1benzopyran]-6-carboxylate de méthyle (317 mg, 0,74 mmol), de soude (60 mg, 1,5 mmol) et d'hydroxyde de lithium (60 mg, 1,5 mmol) dans le THF (5 ml) en présence d'eau et de méthanol est agitée 2 j à température ambiante. Le milieu réactionnel est concentré par distillation à l' évaporateur rotatif sous vide. On rajout 3 ml d'eau et on acidifie à pH=1, par une solution d' acide chlorhydrique concentré. Après filtration, le solide obtenu est lavé à l'hexane et séchée en étuve.
Solide jaune pâle. Masse: 266 mg, Rendement: 86%.Pf: 250 -253°C.
RMN ¹H (DMSO, 250 MHz): 1,70 (6H, s), 2,01 (9H, s), 3,81 (3H, s), 5,15 (2H, s), 6,72 (1H, s), 6,75 (1H Ar, d, J=8,5 Hz), 6,85 à 6,87 (2H, m), 7,14 (1H Ar, d, J=8,5 Hz), 7,74 à 7,78 (2H Ar, m).
RMN ¹³C (DMSO, 250 MHz): 29,04, 37,01, 37,06, 40,50, 54,91, 67,59, 107,93, 115,13, 116,98, 118,75, 122,28, 124,04, 126,78, 128,86, 131,15,131,88, 134,82, 139,17, 157,08, 159,05, 168,17.

### EXEMPLE 10

### 3-[(3,5-Di-tert-butyl-4-hdroxy phenyl)-2H-1-benzopyran]-7-carboxylate de méthyle

### 3-(3,5-Di-tert-butyl-4-hydroxy phenyl)-6-iodo-2H-1-benzopyran

On chauffe 1,5h à reflux une suspension de bromure de 2-hydroxy-4-iodobenzyl triphénylphosphonium (4 g, 6,9 mmol) de carbonate de potassium (1,64 g, 11,9 mmol) et de 3,5-di-tert.butyl-4-hydroxy bromoacétophénone dans le dioxane (80 ml). La solution est refroidie à température ambiante puis, on additionne goutte à goutte une solution de méthanolate de sodium à 30% dans le méthanol (3,36 ml, 17,64 mmol). On chauffe six heures à reflux. Le milieu réactionnel est concentré à I' évaporateur rotatif sous vide à 40°C. On ajoute 60 ml d'eau, puis on acidifie par une solution d'acide chlorhydrique concentré jusqu'à pH=1. On extrait par 60 ml d'éther éthylique. Après décantation la phase organique est lavée deux fois par 60 ml d' eau, séchée sur sulfate de magnésium anhydre et concentré à l' évaporateur rotatif sous vide à 40°C. Le produit est purifié par chromatographie flash sur colonne de silice (CH₂Cl₂ 30 %, heptane 70%).
Solide blanc. Masse: 2,2 g, Rendement: 48%.Pf: 153°C.
RMN ¹H (CDCI₃, 250 MHz): 1,47 (18H, s), 5,13 (2H, s), 5,36 (1H, s), 6,60 (1H, s), 6,79 (1H Ar, d, J=7,75 Hz), 7,20 à 7,24 (4H Ar, m).
RMN ¹³C (CDCl₃, 250 MHz): 30,22, 34,47, 67,54, 91,82, 117,16, 121,81, 123,03, 124,39, 127,71, 130,60, 133,51, 136,20, 153,51, 154,29.

### 3-[(3,5-Di-tert-butyl-4-hydroxy phenyl)-2H-1-benzopyran]-7-carboxylate de méthyle

On chauffe 8h à 80°C une solution de 3-(3,5-Di-tert-butyl-4-hydroxy phenyl)-6-iodo-2H-1-benzopyran (2g, 4,3 mmol), de diacétate de palladium (97 mg, 0,43 mmol), de tributylamine (2,06 ml, 8,6 mmol), dans le méthanol (40 ml) sous une pression de 2,5 bars de monoxyde de carbone. Le milieu réactionnel est concentré à l'évaporateur rotatif sous vide à 40°C. On ajoute 200 ml d'acétate d'éthyle et d'eau. La phase organique est lavée deux fois à l'eau (200 ml), séchée sur sulfate de magnésium anhydre et concentrée à l'évaporateur rotatif sous vide à 40°C. Le produit est purifié par chromatographie flash sur colonne de silice (CH₂Cl₂ 70 %, heptane 30%).
Solide blanc. Masse: 2,2 g, Rendement: 48%.Pf: 153°C.
RMN ¹H (DMSO, 250 MHz): 1,43 (18H, s), 3,84 (3H, s), 5,21 (2H, s), 6,88 (1H, s), 7,14 à 7,31 (4H Ar, m), 7,51 (1H Ar, d, J=8,5 Hz),.
RMN ¹³C (CDCl₃, 250 MHz): 30,16, 34,59, 51,89, 66,75, 115,24, 116,14, 121,45, 122,55, 126,48, 126,77, 127,76, 128,96, 135,64, 139,01, 152,23, 154,86, 165,67.

### EXEMPLE 11

### acide-3-[(3,5-Di-tert-butyl-4-hydroxyphenyl)-2H-1-benzopyran]-7-carboxylique.

Une solution de 3-[(3,5-Di-tert-butyl-4-hydroxyphenyl)-2H-1-benzopyran]-7-carboxylate de méthyle (900 mg, 2,28 mmol), de méthane thiolate (660 mg, 9,4 mmol) dans la diméthyl formamide (30 ml) est chauffée à 100°C pendant 4h. On ajoute 20 ml d'eau, 20 ml d'éther éthylique puis on acidifie à pH=1, par une solution d' acide chlorhydrique concentré. La phase organique est lavée deux fois à l'eau, séchée sur sulfate de magnésium anhydre et concentrée à l'évaporateur rotatif sous vide à 40°C. Le produit est purifié par chromatographie flash sur colonne de silice (acétate d' éthyle 40 %, heptane 60 %).
Solide blanc. Masse: 775 mg, Rendement: 89%.Pf: 215°C.
RMN ¹H (CDCl₃, 250 MHz): 1,48 (18H, s), 5,21 (2H, s), 5,41 (1H, s), 6,71 (1H, s), 7,15 (1H Ar, d, J=8Hz), 7,29 (2H Ar, s), 7,56 (1H Ar, s), 7,51 (1H Ar, d, J=8 Hz),.
RMN ¹³C (CDCl₃, 250 MHz): 29,80, 34,07, 67,09, 116,48, 116,74, 121,60, 123,38, 125,88, 127,00, 128,23, 128,39, 135,82, 135,88, 152,31, 154,24, 171,19.

### EXEMPLE 12

### 3-[(3-(1-adamantyl)-4-hydroxyphényl)-2H-1-benzopyran]-7-carboxylate de méthyl.

3-(1-Adamantyl)-4-hydroxybromoacétophènone.

La 3-adamantyl 4-hydroxyacétophénone (2,5 g, 9,25 mmol) est dissoute dans 15 ml de dioxane et 15 ml d' éther éthylique. Une solution de brome (2,3ml, 10,4 mmol) dans le dichlorométhane (5 ml), est additionné goutte à goutte. On agite une heure à température ambiante. Le milieu réactionnel est versé dans 100 ml d' un mélange eau et glace. Après agitation et décantation, la phase organique est lavée deux fois par 50 ml d' eau, séchée sur sulfate de magnésium et concentrée à l' évaporateur rotatif sous vide à 40 °C.
Solide rose. Masse: 1,8 g. Rendement: 56 %.
RMN ¹H (CDCL₃, 250 MHz): 1,78 (6H, s), 2,12 (9H, s), 4,40 (2H, s), 6,09 (1H Ar, s), 6,75 (1H Ar, d, J=8,25 Hz), 7,74 (1H Ar, dd, J1 =8,25 Hz, J2=2 Hz), 7,93 (1H Ar, d, J=2 Hz).

### Acide 2-hydroxy-4-iodobenzoique

A une solution d'acide sulfurique à 20 % (650 ml), on ajoute de l'acide 4-amino-2-hydroxybenzoique et 200ml d'une solution d'acide sulfurique à 20 %. La solution est refoidie à -10°C et une solution de nitrite de sodium (47g, 0,34 mol) dans l'eau (100ml), est additionnée en 5h. La solution obtenue est additionnée goutte à goutte sur une suspension d'iodure de potassium (69,5g, 0,42 mol) et d'iodure de cuivre (I) (69,5 g, 0,36 mol) dans 370 ml d'acide sulfurique à 20 %. On agite 36 h à température ambiante, puis on filtre. Le filtrat est extrait à l'acétate d'éthyle lavé deux fois par une solution saturée de sulfite de sodium et deux fois à l'eau. La phase organique est concentrée à l'évaporateur rotatif sous vide à 40°C.
Solide blanc. Masse: 24,25 g, Rendement: 35%.Pf: 192°C.
RMN ¹H (DMSO, 250 MHz): 7,17 (1H Ar, d, J=8,25 Hz), 7,25 (1H Ar, s), 7,42 (1H Ar, d, J=8,25 Hz).

### Alcool 2-hydroxy-4-iodobenzylique

On additionne goutte à goutte à 0°C une solution 1M d'hydrure de bore dans le THF, à une solution d'acide 2-hydroxy-4-iodobenzoique (13,2g, 0,05 mol) dans le THF (100 ml). On agite 6h à température ambiante puis 20 ml d'une solution de THF et d'eau (1:1) sont additionnés. Après concentration à l'évaporateur rotatif sous vide à 40°C, on extrait à l'acétate d'éthyle. La phase organique est lavée à l'eau, séchée sur sulfate de magnésium anhydre, concentrée à l'évaporateur rotatif sous vide à 40°C. Le produit est purifié par chromatographie flash sur colonne de silice (AcOEt 60 %, heptane 40%).
Solide blanc. Masse: 11 g, Rendement: 88%.
RMN ¹H (CDCl₃, 250 MHz): 4,50 (2H, s), 6,81 (1H Ar, d, J=7,75 Hz), 6,99 (1H Ar, d, J=7,75 Hz), 7,05 (1H Ar, s), 9,06 (1H, s).

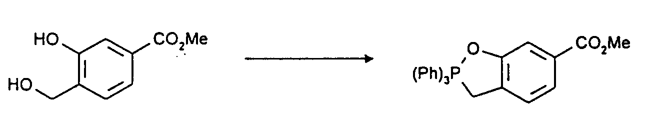

3-hydroxy-4-hydroxyméthyl benzoate de méthyl.

On chauffe 5h à 80°C sous pression de monoxyde de carbone (2,5 bars), une solution d'Alcool 2-hydroxy-4-iodobenzylique (1,25 g, 5 mmol), de diacétate de palladium (55 mg, 0,5 mmol), de triéthylamine (1,4 ml, 10 mmol) dans le méthanol (50 ml). Après concentration à l'évaporateur sous vide à 40°C l'huile obtenue est diluée dans le dichlorométhane (20 ml) et lavée trois fois avec 20 ml d'eau. Le produit est purifié par chromatographie flash sur colonne de silice (AcOEt 30 %, CH₂Cl₂ 30%).
Solide blanc. Masse: 640 mg. Rendement: 70%.
RMN ¹H (CDCl₃, 250 MHz): 3,90 (3H, s), 4,90 (2H, s), 7,11 (1H Ar, d, J=8 Hz), 6,99 7,51 (1H Ar, d, J=8 Hz), 7,53 (1H Ar, s).

### Bromure de 2-hydroxy-4-méthoxycarbonyl benzyl triphénylphosphonium

On chauffe à reflux pendant 1,5 h une suspension de bromhydrate de triphénylphosphine (11,9 g, 34,7 mmol), de 3-hydroxy-4-hydroxyméthyl benzoate de méthyl (6g, 35 mmol) dans l'acétonitrile (65 ml). Le solvant est éliminé à l'évaporateur rotatif sous vide et le solide résultant est dilué dans l'éther éthylique (30 ml). Le produit est isolé par filtration.
Solide blanc. Masse: 16,3 g. Rendement: 91%.
RMN ¹H (CDCl₃, 250 MHz): 3,70 (3H, s), 5,01 (2H, d, J=16,25 Hz), 7,01 (1H Ar, d, J=8 Hz), 7,23 (1H Ar, d, J=8 Hz), 7,40 (1H Ar, s), 7,59 à 7,92 (15H Ar, m).

### 5-méthoxycarbonyl-2,2,2(3H)-triphényl-1,2-benzoxaphosphole

A une solution de Bromure de 2-hydroxy-4-méthoxycarbonyl-benzyl triphénylphosphonium (2g, 3,94 mmol) dans 44 ml d'eau on ajoute, à température ambiante 4,4 ml de soude 1N. Après 20 mn d'agitation, le précipité est filtré et séché.
Solide jaunâtre.
RMN ¹H (CDCL₃, 250 MHz): 3,77 (3H, s), 4,60 (2H, d, J=13,5 Hz), 6,90 (1H Ar, d, J=7,75 Hz), 7,07 (1H Ar, d, J=7,75 Hz), 7,34 (1H Ar, s), 7,48 à 7,71 (15 H Ar, m).

3-[(3-(1-adamantyl)-4-hydroxyphényl)-2H-1-benzopyran]-7-carboxylate de méthyl.

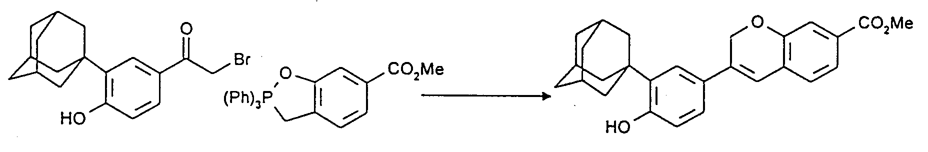

On agite 1h à température ambiante une solution de 5-carboxylate de méthyl-2,2,2(3H)-triphényl-1,2-benzoxaphosphole (1,68 g, 3,94 mmol) et de 3-(1-Adamantyl)-4-hydroxybromoacétophénone (1,05 g, 3 mmol) dans le dichlorométhane. Après concentration, 15 ml de dioxane sont ajoutés. La solution est chauffée à reflux puis une solution de méthanolate de sodium à 30 % dans le méthanol (0,6 ml, 3,15 mmol) est additionnée goutte à goutte. On poursuit le chauffage pendant 3h puis le milieu réactionnel est concentré à l' évaporateur rotatif sous vide à 40°C. On ajoute 40 ml d'eau, puis on extrait par 40 ml d'éther éthylique. Après décantation la phase organique est lavée deux fois par 40 ml d' eau, séchée sur sulfate de magnésium anhydre et concentré à l' évaporateur rotatif sous vide à 40°C. Le produit est purifié par cristallisation dans un mélange dichlorométhane heptane (2:8).
Solide jaune pâle. Masse: 520 mg, Rendement: 41,5%.Pf: 205°C.
RMN ¹H (DMSO, 250 MHz): 1,74 (6H, s), 2,05 (3H, s), 2,11 (6H, s), 3,83 (3H, s), 5,20 (2H, s), 6,83 (1H Ar, d, J=8,25 Hz), 6,94 (1H, s), 7,23 à 7,29 (4H Ar, m), 7,51 (1H Ar, d, J=8,25 Hz), 9,70 (1H, s).
RMN¹³C (DMSO, 250 MHz): 28,32, 36,30, 36,54, 39,65, 51,96, 66,57, 115,06, 115,26, 116,49, 122,55, 123,15, 123,54, 125,79, 126,49, 127,87, 128,76, 135,14, 135,82, 152,14, 157,06, 165,68.

### EXEMPLE 13

### acide 3-[(3-(1-adamantyl)-4-hydroxyphényl)-2H-1-benzopyran]-7-carboxylique.

On chauffe à reflux pendant 4 h une solution de 3-[(3-(1-adamantyl)-4-hydroxyphényl)-2H-1-benzopyran]-7-carboxylate de méthyl (115 mg, 0,27 mmol), d'hydroxyde de lithium (50 mg, 1,25 mmol), d'hydroxyde de sodium (50 mg, 1,25 mmol) dans 5 ml d'un mélange THF, méthanol et eau (5, 1, 1). Après concentration à l'évaporateur rotatif sous vide à 40°C, on ajoute 5 ml d'eau et 5 ml d'acétate d'éthyle. On acidifie jusqu'à pH 1 par une solution d'acide chlorhydrique concentré. Après décantation la phase organique est lavée deux fois à l'eau, séchée sur sulfate de magnésium anhydre et concentrée à l'évaporateur rotatif sous vide à 40°C.
Solide jaune pâle. Masse: 88 mg, Rendement: 79%.Pf: 293-297°C.
RMN ¹H (DMSO, 250 MHz): 1,58 (6H, s), 1,89 (3H, s), 1,95 (6H, s), 5,03 (2H, s), 6,66 (1H Ar, d, J=8,25 Hz), 6,78 (1H, s), 7,08 à 7,11 (4H Ar, m), 7,33 (1H Ar, d, J=8,25 Hz), 9,53 (1H, s), 12,70 (1H, s).
RMN¹³C (DMSO, 250 MHz): 28,35, 36,35, 36,58, 39,70, 66,57, 115,32, 115,45, 116,54, 122,75, 123,16, 123,55, 125,93, 126,41, 127,51, 130,11, 134,88, 135,87, 152,12, 157,03, 166,80.

### EXEMPLE 14

### 3-[(3-tert-butyl-4-hdroxy phenyl)-2H-1-benzopyran]-7-carboxylate de méthyle

On agite 30 mn à température ambiante une solution de 5-carboxylate de méthyl-2,2,2(3H)-triphényl-1,2-benzoxaphosphole (203 mg, 0,75 mmol) et de 3-tert.butyl-4-hydroxy bromoacétophénone (305 mg, 0,72 mmol) dans le dichlorométhane (3ml). Après concentration on ajoute 3 ml de dioxane. La solution est chauffée à reflux puis une solution de méthanolate de sodium à 30 % dans le méthanol (0,15 ml, 0,78 mmol) est additionnée goutte à goutte. On poursuit le chauffage pendant 3h puis le milieu réactionnel est concentré à l' évaporateur rotatif sous vide à 40°C. On ajoute 10 ml d'eau, puis on extrait par 10 ml d'éther éthylique. Après décantation la phase organique est lavée deux fois par 10 ml d' eau, séchée sur sulfate de magnésium anhydre et concentré à l' évaporateur rotatif sous vide à 40°C. Le produit est purifié par chromatographie flash sur colonne de silice (dichlorométhane).
Solide blanc. Masse: 460 mg, Rendement: 51%.Pf: 159°C.
RMN ¹H (CDCI₃, 250 MHz): 1,44 (9H, s), 3,90 (3H, s), 5,17 (2H, s), 5,25 (1H, s), 6,69 à 6,73 (2H, m), 7,10 (1H Ar, d, J=7,75Hz), 7,15 (1H Ar, dd, J1=8,25Hz, J2=2,25Hz), 7,26 (1H Ar, s), 7,39 (1HAr, d, J=1,75Hz), 7,49 (1H Ar, s), 7,60 (1H Ar, d, J=7,75Hz).
RMN ¹³C (CDCl₃, 250 MHz): 29,28, 34,56, 51,91, 67,18, 116,19, 116,69, 117,08, 122,93, 123,57, 123,83, 126,10, 127,51, 128,32, 129,60, 134,75, 136,41, 152,52, 154,75, 166,66.

### EXEMPLE 15

### Acide-3-[(3-tert-butyl-4-hydroxy phenyl)-2H-1-benzopyran]-7-carboxylique.

On chauffe à reflux pendant 3 h une solution de 3-[(3-tert-butyl-4-hydroxy phenyl)-2H-1-benzopyran]-7-carboxylate de méthyle (100 mg, 0,3 mmol), d'hydroxyde de lithium (50 mg, 2 mmol), d'hydroxyde de sodium (50 mg, 1,25 mmol) dans 10 ml d'un mélange THF, méthanol et eau (5, 1, 1). Après concentration à l'évaporateur rotatif sous vide à 40°C, on ajoute 5 ml d'eau et 5 ml d'acétate d'éthyle. On acidifie jusqu'à ph 1 par une solution d'acide chlorhydryque concentré. Après décantation la phase organique est lavée deux fois à l'eau, séchée sur sulfate de magnésium anhydre et concentrée à l'évaporateur rotatif sous vide à 40°C.
Solide blanc. Masse: 85 mg, Rendement: 87%.Pf: 208°C.
RMN ¹H (CDCI₃, 250 MHz): 1,44 (9H, s), 3,90 (3H, s), 5,17 (2H, s), 6,71 (1H, s), 6,85 (1H Ar, d, J=8,5Hz), 7,08 à 7,15 (2H Ar, m), 7,37 (1H Ar, s), 7,49 (1H Ar, s), 7,61 (1H Ar, d, J=7,75Hz) 8,61 (1H, s).
RMN ¹³C (CDCl₃, 250 MHz): 29,20, 34,59, 67,17, 116,30, 116,57, 123,14, 123,38, 125,88, 126,75, 127,58, 130,01, 135,00, 136,30, 152,37, 156,51, 168,30.
RMN ¹H (DMSO, 250 MHz): 1,43 (18H, s), 3,84 (3H, s), 5,21 (2H, s), 6,88 (1H, s), 7,14 à 7,31 (4H Ar, m), 7,51 (1H Ar, d, J=8,5 Hz),.

### EXEMPLE 16

### 3-[(3-(1-adamantyl)-4-méthoxyethoxyméthoxyphényl)-2H-1 benzopyran]-7-carboxylate de méthyl.

On agite 1h à 0°C une solution de 3-[(3-(1-adamantyl)-4-hydroxyphényl)-2H-1-benzopyran]-7-carboxylate de méthyl (165 mg, 0,4 mmol) et d'hydrure de sodium à 80% (14 mg, 0,46 mmol) dans le DMF (2 ml). On additionne à 0°C, goutte à goutte le chlorure de méthoxyéthoxyméthyl (0,054 ml, 0,47 mmol). On agite 12h à température ambiante. On ajoute 10 ml d'eau et 10 ml d'acétate d'éthyle. Le pH est ajusté à 1 par une solution d'acide chlorhydryque concentrée. La phase organique est lavée deux fois à l'eau, séchée sur sulfate de magnesium anhydre et concentrée à l'évaporateur rotatif sous vide à 40°C. Le produit est purifié par chromatographie flash sur colonne de silice (CH₂Cl₂ 97 %, éther éthylique 3%, Rf: 0,52).
Solide jaunâtre. Masse: 135 mg. Rendement: 67 %.
RMN ¹H (CDCL₃, 250 MHz): 1,79 (6H, s), 2,13 (9H, s), 3,40 (3H, s), 3,59 (2H, t, J=4,5 Hz), 3,86 (2H, t, J=4,5 Hz), 3,90 (3H, s), 5,19 (2H, s), 5,35 (2H, s), 6,74 (1H, s), 7,11 (1H Ar, d, J=7,75 Hz), 7,19 à 7,21 (2H Ar, m), 7,37 (1H Ar, s), 7,48 (1H, s), 7,59 (1H Ar, d, J=7,75 Hz).
RMN¹³C (CDCL₃, 250 MHz): 29,07, 37,07, 37,32, 40,67, 52,08, 59,09, 67,40, 67,97, 71,62, 93,33, 114,83, 116,41, 117,59, 123,11, 123,65, 126,35, 127,66, 129,31, 129,94, 134,98, 138,97, 152,79, 157,01, 166,74.

### EXEMPLE 17

### acide 3-[(3-(1-adamantyl)-4-méthoxyethoxyméthoxyphényl)-2H-1-benzopyran]-7-carboxylique.

On chauffe à reflux pendant 5 h une solution de 3-[(3-(1-adamantyl)-4-méthoxyethoxyméthoxyphényl)-2H-1-benzopyran]-7-carboxylate de méthyl (100 mg, 0,2 mmol), d'hydroxyde de lithium (50 mg, 1,25 mmol), d'hydroxyde de sodium (50 mg, 1,25 mmol) dans 5 ml d'un mélange THF, méthanol et eau (5, 1, 1). Après concentration à l'évaporateur rotatif sous vide à 40°C, on ajoute 5 ml d'eau et 5 ml d'acétate d'éthyle. On acidifie jusqu'à ph 1 par une solution d'acide chlorhydryque concentré. Après décantation la phase organique est lavée dux fois à l'eau, séchée sur sulfate de magnésium anhydre et concentrée à l'évaporateur rotatif sous vide à 40°C.
Solide jaune pâle. Masse: 80 mg, Rendement: 82%.Pf: 203-204°C.
RMN ¹H (DMSO, 250 MHz): 1,90 (6H, s), 2,14 (3H, s), 2,22 (6H, s), 3,39 (3H, s), 3,65 (2H, t, J=4,75 Hz), 3,93 (2H, t, J=4,75 Hz), 5,37 (2H, s), 5,49 (2H, s), 7,17 6,78 (1H, s), 7,25 (1H Ar, d, J=9 Hz), 7,43 à 7,45 (2H Ar, m), 7,50 à 7,53 (2H Ar, m),7,65 (1H Ar, d, J=9 Hz), 13,01 (1H, s).
RMN¹³C (DMSO, 250 MHz): 28,49, 36,60, 36,83, 40,13, 58,16, 66,65, 67,97, 71,08, 93,02, 114,63, 115,52, 116,85, 122,88, 123,34, 123,92, 126,79, 127,39, 128,35, 130,58, 134,55, 138,14, 152,38, 156,48, 166,90.

### EXEMPLES 18 ET 19

### 3-[(5,6,7,8-tétrahydro-5,5,8,8-tétraméthyl-2-naphtyl)-2H-1-benzopyran]-7-méthyl alcohol et 3-[(5,6,7,8-tétrahydro-5,5,8,8-tétraméthyl-2-naphtyl)-2H-1-benzopyran]-7-carboxaldéhyde.

### 5-lodo-2,2,2(3H)-triphényl-1,2-benzoxaphosphole

A une solution de Bromure de 2-hydroxy-4-iodo benzyl triphénylphosphonium (4g, 6,95 mmol) dans 75 ml d'eau on ajoute, à température ambiante 7,8 ml de soude 1N. Après 30 mn d'agitation, le précipité est filtré et séché.
Solide jaunâtre.

7-iodo-3-(5,6,7,8-tétrahydro-5,5,8,8-tétraméthyl-2-naphtyl)-2H-1-benzopyran.

On agite 1h à température ambiante une solution de 5-iodo-2,2,2(3H)-triphényl-1,2-benzoxaphosphole (3,43 g, 6,95 mmol) et de 5,6,7,8-tétrahydro-5,5,8,8-tétraméthylbromoacétonaphtone (2,65g, 8,6 mmol) dans le dichlorométhane. Après concentration, 25 ml de dioxane sont ajoutés. La solution est chauffée à reflux puis une solution de méthanolate de sodium à 30 % dans le méthanol (1,45 ml, 7,6 mmol) est additionnée goutte à goutte. On poursuit le chauffage pendant 3h puis le milieu réactionnel est concentré à l' évaporateur rotatif sous vide à 40°C. On ajoute 80 ml d'eau, puis on extrait par 80 ml d'éther éthylique. Après décantation la phase organique est lavée deux fois par 80 ml d' eau, séchée sur sulfate de magnésium anhydre et concentré à l' évaporateur rotatif sous vide à 40°C. Le produit est purifié par chromatographie flash (CH₂CL₂ 80 %, hexane 20 %)(Rf: 0,37).
Solide jaune pâle. Masse: 2,03 g, Rendement: 66%.Pf: 132°C.
RMN ¹H (CDCl₃, 250 MHz): 1,29 (6H, s), 1,31 (6H, s), 1,70 (4H, s), 5,14 (2H, s), 6,68 (1H, s), 6,79 (1H Ar, d, J=7,75 Hz), 7,17 à 7,35 (5H Ar, m).
RMN¹³C (DMSO, 250 MHz): 32,19, 32,33, 34,73, 34,84, 35,37, 35,52, 67,85, 92,76, 118,82, 122,66, 123,23, 123,32, 124,95, 127,48, 128,37, 131,10, 133,25, 134,00, 145,81, 146,00, 154,15.

3-[(5 6,7,8-tétrahydro-5,5,8,8-tétraméthyl-2-naphtyl)-2H-1-benzopyran]-7-méthyl alcohol.
3-[(5,6,7,8-tétrahydro-5,5,8,8-tétraméthyl-2-naphtyl)-2H-1-benzopyran]-7-carboxaldéhyde.
Acide 3-[(5,6,7,8-tétrahydro-5,5,8,8-tétraméthylnaphtyl)-2H-1-benzopyran]-7-carboxylique.

A -70°C on additionne une solution de buthyl lithium 2,5 M dans l'hexane (1ml, 2,48 mmol) à une solution de 7-iodo-3-(5,6,7,8-tétrahydro-5,5,8,8-tétraméthyl-2-naphtyl)-2H-1-benzopyran (1 g, 2,25 mmol) dans le THF (10 ml). On agite à70°C pendant 45 mn. On laisse remonter à -45°C et on additionne 0,2 ml de DMF (2,6 mmol) goutte à goutte. On agite à température ambiante 12 h. On ajoute 40 ml d'éther et 40 ml d'eau et on agite 1h à température ambiante. Après décantation, la phase organique est lavée deux fois par 40 ml d'eau. L'acide et l'alcool sont séparés de l'aldéhyde par cristallisation dans le dichlorométhane. L'acide est séparé de l'alcool par cristallisation dans l'éther éthylique. Les trois produits sont lavés à l'heptane.

3-[(5,6,7,8-tétrahydro-5,5,8,8-tétraméthyl-2-naphtyl)-2H-1-benzopyran]-7-carboxaldéhyde.
Solide jaune. Masse: 42 mg, Rendement: 5,4%.Pf: 140°C.
RMN ¹H (CDCl₃, 250 MHz): 1,30 (6H, s), 1,32 (6H, s), 1,71 (4H, s), 5,23 (2H, s), 6,79 (1H, s), 7,19 à 7,24 (2H Ar, m), 7,32 à 7,44 (4H Ar, m), 9,89 (1H, s) . RMN ¹³C (CDCl₃, 250 MHz): 31,73; 31,88; 34,35; 34,41; 34,88; 35,03; 67,42; 100,78; 115,561; 118,31; 122,37; 123,09; 124,00; 127,06; 127,15; 129,09; 133,12; 136,00; 136,71; 145,51; 146,25; 153,53; 191,35.

3-[(5,6,7,8-tétrahydro-5,5,8,8-tétraméthyl-2-naphtyl)-2H-1-benzopyran]-7-méthyl alcohol.
Solide blanc. Masse: 118 g, Rendement: 15%.Pf: 126°C.
RMN ¹H (CDCl₃, 250 MHz): 1,30 (6H, s), 1,33 (6H, s), 1,71 (4H, s), 4,64 (2H, s), 5,30 (2H, s), 6,76 (1H, s), 6,87 à 6,93 (2H Ar, m), 7,09 (1H Ar, d, J=7,5 Hz), 7,22 (1H Ar, d, J=8,25 Hz), 7,33 à 7,38 (2H Ar, m).
RMN ¹³C (CDCl₃, 250 MHz): 31,49; 31,62; 33,99; 34,12; 34,69; 34,83; 64,94; 67,09; 113,72; 118,64; 119,76; 121,95; 122,36; 122,58; 126,68; 126,71; 131,94; 133,59; 141,65; 144,99; 145,03; 153,20.

Acide 3-[(5,6,7,8-tétrahydro-5,5,8,8-tétraméthylnaphtyl)-2H-1-benzopyran-7-yl] carboxylique.
Solide blanc. Masse: 103 mg, Rendement: 13%. Pf: 285°C
RMN ¹H (DMSO, 250 MHz): 1,15 (6H, s), 1,2 (6H, s), 1,56 (4H, s), 5,15 (2H, s), 6,99 (1H, s), 7,24 (4H Ar, m), 7,41 (2H Ar, m), 12,78 (1H, s) .
¹³C (DMSO, 250 MHz): 31,64, 31,75, 34,15, 34,28, 34,65, 34,89, 66,76, 115,73, 117,98, 122,65, 123,02, 123,15, 127,06, 127,37, 130,96, 132,92, 134,77, 145,06, 145,47, 152,71, 167,01.

### EXEMPLE 20

### 3-[(3,5-Di-tert-butyl-4-hydroxy phenyl)-2H-1-benzopyran]-7-carboxanilide

On chauffe 8 h à 100°C une solution de 3-(3,5-Di-tert-butyl-4-hydroxy phenyl)-6-iodo-2H-1-benzopyran (250 mg, 0,54 mmol), de chlorure de bis (triphenylphosphine) palladium (II) (38 mg, 0,05 mmol), d'aniline (94 ul, 1,1 mmol) dans la tributylamine (40 ml) sous une pression de 2,5 bars de monoxyde de carbone. Le milieu réactionnel est traité par de l'eau et de l'éther éthylique. La phase organique est lavée par une solution d'acide chlorhydrique 2N puis à l'eau, séchée sur sulfate de magnésium anhydre et concentrée à l'évaporateur rotatif sous vide à 40°C. Le produit est purifié par chromatographie flash sur colonne de silice (CH₂Cl₂).
Solide blanc. Masse: 45 mg, Rendement: 12%.Pf: 50°C.
RMN ¹H (CDCl₃, 250 MHz): 1,41 (18H, s), 5,13 (2H, s), 5,33 (1H, s), 6,63 (1H, s), 7,09 (2H Ar, d, J=7,75Hz), 7,21 à 7,36 (5H Ar, m), 7,57 (2H Ar, d, J=7,75 Hz), 7,75 (1H Ar, s).
RMN ¹³C (CDCl₃, 250 MHz): 30,61, 34,87, 67,97, 114,40, 117,41, 120,56, 120,80, 122,31, 124,80, 127,01, 127,16, 127,39, 127,84, 129,43, 132,90, 134,97, 135,63, 136,68, 138,42, 153,46, 154,94, 165,53.

### EXEMPLES 21 et 22

### Morpholide de l'acide 3-[(5,6,7,8-tétrahydro-5,5,8,8-tétraméthylnapthtyl)-2H-1-benzopyran]-7-carboxylique et Morpholide de l'acide 3-[(5,6,7,8-tétrahydro-5,5,8,8-tétraméthylnaphtyl)-2H-1-benzopyran]-7-glyoxylique.

On chauffe 4 h à 100°C une solution de 7-iodo-3-(5,6,7,8-tétrahydro-5,5,8,8-tétraméthyl-2-naphtyl)-2H-1-benzopyran (400 mg, 0,88 mmol), de chlorure de bis (triphenylphosphine) palladium (II) (62 mg, 0,09 mmol) dans la morpholine (40 ml) sous une pression de 2,5 bars de monoxyde de carbone. Le milieu réactionnel est traité par de l'eau et de l'acétate d'éthyle. La phase organique est lavée par une solution d'acide chlorhydrique 2N puis à l'eau, séchée sur sulfate de magnésium anhydre et concentrée à l'évaporateur rotatif sous vide à 40°C. Les produits sont séparés par chromatographie flash sur colonne de silice (AcOEt 4, heptane 6).
Solide jaune. Masse: 330mg, Rendement: 78%.Pf: 162°C

Morpholide de l'acide 3-[(5,6,7,8-tétrahydro-5,5,8,8-tétraméthylnaphtyl)-2H-1-benzopyran]-7-carboxylique.
RMN ¹H (CDCl₃, 250 MHz): 1,22 (6H, s), 1,25 (6H, s), 1,63 (4H, s), 3,31 à 3,70 (8H, m), 5,21 (2H, s), 6,83 (1H, s), 6,93 (1H Ar, d), 7,03 (1H Ar, s), 7,23 à 7,38 (3H Ar, m), 7,48 (1H Ar, s).

Morpholide de l'acide 3-[(5,6,7,8-tétrahydro-5,5,8,8-tétraméthylnaphtyl)-2H-1-benzopyran]-7-glyoxylique.
Solide jaune. Masse: 50mg.Pf: 140°C
RMN ¹H (CDCl₃, 250 MHz): 1,22 (6H, s), 1,25 (6H, s), 1,63 (4H, s), 3,31 (2H, t, J=5Hz), 3,59 (2H, t, J=5Hz), 3,71 (4H, s), 5,16 (2H, s), 6,79 (1H, s), 7,17 à 7,26 (3H Ar, m), 7,30 à 7,31 (2H Ar, m), 7,42 (1H Ar, d, J1=1,5Hz, J2=7,75Hz).

### EXEMPLE 23

### N-butyl-3-[(5,6,7,8-tétrahydro-5,5,8,8-tétraméthylnaphtyl)-2H-1-benzopyran]-7-carboxamide.

On procède selon le même mode opératoire que les exemples 23 et 24 en remplaçant la morpholine par la N-butylamine.
Solide jaune. Masse: 33 mg
RMN ¹H (CDCl₃, 250 MHz): 0,96 (3H, t), 1,30 (6H, s), 1,32 (6H, s), 1,32 à 1,48 (2H, m), 1,54 à 1,66 (2H, m), 1,71 (4H, s), 3,39 (2H, q), 5,13 (2H, s), 6,80 (1H, s), 7,03 à 7,58 (6H, m), 7,78 (1H Ar, s), 8,00 (1H Ar, d).

### EXEMPLE 24

### Activites biologiques

| **EXEMPLES** | **F9 AC** _{**50**} **(nM)**^{**a**} | **Transactivation RAR AC**_{**50**} **(nM)**^{**b**} | | | **Binding RAR Kd (nM)**^{**c**} | | |
|---|---|---|---|---|---|---|---|
| | | **RAR α** | **RAR β** | **RAR γ** | **RAR α** | **RAR β** | **RAR γ** |
| 1 | 34 | 8 | 2 | 4 | 764 | 71 | 174 |
| 13 | 107 | 47 | 20 | 6 | 821 | 3296 | 148 |
| 11 | 320 | 124 | 77 | 157 | 1471 | 3678 | 531 |
| 2 | 58 | 22 | 2 | 15 | 487 | 36 | 19 |

a) Après traitement par les produits cités, les cellules F9 de tératocarcinome embryonaire de souris se différencient en cellules endodermiques. Cette différenciation est caractérisée par la sécrétion dans le milieu de culture de l'activateur du plasminogène. L'activité du produit est exprimée par la valeur d'AC₅₀ qui représente la concentration du produit testé que produit la moitié de la quantité maximale d'activateur de plasminogène sécrété.
b) Ce test est effectué comme décrit dans la publication: Bernard B.A., Bernardon J.M., Delescluse C., Martin C., Lenoir M.C., Maignan J., Carpentier B., Pilgrim W.R., Reichert U. and Shroot B. Biochem. Biophys. Res. Comm. (1992), 186, 977-983. Des cellules HeLa sont cotransfectées avec des vecteurs d'expressions codant pour les RARs humains et le plasmide rapporteur TREpp-tk-CAT. La valeur donnée représente la concentration du produit testé qui donne 50% de l'activité CAT maximale.
c) Le test de binding est effectué sur des récepteurs humains recombinants produits par la transfection de cellules cos-7 avec des vecteurs d'expression codant les sous types de récepteurs RARs. Les extraits nucléaires obtenus à partir des cellules transfectées sont utilisés dans des expériences de compétition avec l'acide 4-(5,6,7,8-tétrahydro-5,5,8,8-tétraméthyl-2-anthacényl)benzoïque marqué au tritium (décrit dans le brevet EP-B-0.359.621, nommé le [H³]-CD 367).

### B. EXEMPLES DE FORMULATION

### 1) VOIE ORALE

(a) On prépare la composition suivante sous la forme d'un comprimé de 0,8 g

| | |
|---|---|
| Composé de l'exemple 2 | 0,005 g |
| Amidon prégélatinisé | 0,265 g |
| Cellulose microcristalline | 0,300 g |
| Lactose | 0,200 g |
| Stéarate de magnésium | 0,030 g |

Pour le traitement de l'acné, on administre à un individu adulte 1 à 3 comprimés par jour pendant 3 à 6 mois selon la gravité du cas traité.
(b) On prépare une suspension buvable, destinée à être conditionnée en ampoules de 5 ml

| | |
|---|---|
| Composé de l'exemple 1 | 0,050 g |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p. | 5 ml |

Pour le traitement de l'acné, on administre à un individu adulte 1 ampoule par jour pendant 3 mois selon la gravité du cas traité.
(c) On prépare la formulation suivante destinée à être conditionnée en gélules :

| | |
|---|---|
| Composé de l'exemple 3 | 0,025 g |
| Amidon de maïs | 0,060 g |
| Lactose q.s.p. | 0,300 g |

Les gélules utilisées sont constituées de gélatine, d'oxyde de titane et d'un conservateur.

Dans le traitement du psoriasis, on administre à un individu adulte, 1 gélule par jour pendant 30 jours.

### 2) VOIE TOPIQUE

(a) On prépare la crème Eau-dans l'Huile non ionique suivante :

| | |
|---|---|
| Composé de l'exemple 3 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau psoriatique 1 à 2 fois par jour pendant 30 jours.
(b) On prépare un gel en réalisant la formulation suivante :

| | |
|---|---|
| Composé de l'exemple 1 | 0,050 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p. | 100,000 g |

Ce gel est appliqué sur une peau atteinte de dermatose ou une peau acnéique 1 à 3 fois par jour pendant 6 à 12 semaines selon la gravité du cas traité.
(c) On prépare une lotion antiséborrhéique en procédant au mélange des ingrédients suivants :

| | |
|---|---|
| Composé de l'exemple 1 | 0,030 g |
| Propylène glycol | 5,000 g |
| Butylhydroxytoluène | 0,100 g |
| Ethanol (à 95°) q.s.p. | 100,000 g |

Cette lotion est appliquée deux fois par jour sur un cuir chevelu séborrhéique et on constate une amélioration significative dans un délai compris entre 2 et 6 semaines.
(d) On prépare une composition cosmétique contre les effets néfastes du soleil en procédant au mélange des ingrédients suivants :

| | |
|---|---|
| Composé de l'exemple 5 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p. | 100,000 g |

Cette composition est appliquée quotidiennement, elle permet de lutter contre le vieillissement photoinduit.
(e) On prépare la crème Huile dans l'Eau non ionique suivante :

| | |
|---|---|
| Composé de l'exemple 1 | 0,500 g |
| Vitamine D3 | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau psoriatique 1 à 2 fois par jour pendant 30 Jours.
(f) On prépare un gel topique en procédant au mélange des ingrédients suivants :

| | |
|---|---|
| Composé de l'exemple 2 | 0,050 g |
| Ethanol | 43,000 g |
| α -tocophérol | 0,050 g |
| Polymère carboxyvinylique vendu sous la dénomination "Carbopol 941" par la société "Goodrich" | 0,500 g |
| Triéthanolamine en solution aqueuse à 20 % en poids | 3,800 g |
| Eau | 9,300 g |
| Propylène glycol qsp. | 100,000 g |

Ce gel est appliqué dans le traitement de l'acné 1 à 3 fois par jour pendant 6 à 12 semaines selon la gravité du cas traité.
(g) On prépare une lotion capillaire anti-chute et pour la repousse des cheveux en procédant au mélange des ingrédients suivants :

| | |
|---|---|
| Composé de l'exemple 3 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

On applique cette lotion 2 fois par jour pendant 3 mois sur un cuir chevelu ayant subi une chute de cheveu importante.
(h) On prépare une crème anti-acnéique en procédant au mélange des ingrédients suivants :

| | |
|---|---|
| Composé de l'exemple 1 | 0,050 g |
| Acide rétinoïque | 0,010 g |
| Mélange de stéarate de glycérol et de stérarate de polyéthylène glycol (75 moles) vendu sous le nom de "Gelot 64" par la société "GATTEFOSSE" | 15,000 g |
| Huile de palme polyoxyéthylénée à 6 moles d'oxyde d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp | 100,000 g |

Cette crème est appliquée sur une peau atteinte de dermatose ou une peau acnéique 1 à 3 fois par jour pendant 6 à 12 semaines.
(i) On prépare une crème huile dans l'eau en réalisant la formulation suivante :

| | |
|---|---|
| Composé de l'exemple 2 | 0,020 g |
| 17-valérate de bétamethasone | 0,050 g |
| S-carboxyméthyl cystéine | 3, 000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthyléné à 20 moles d'oxyde d'éthyléne vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p. | 100,000 g |

Cette crème est appliquée 2 fois par jour sur une peau atteinte de dermatose pendant 30 jours.
(j) On prépare la crème de type huile dans l'eau suivante :

| | |
|---|---|
| Acide lactique | 5,000 g |
| Composé de l'exemple 2 | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthyléné à 20 moles d'oxyde d'éthylène vendu sous le nom de "Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" ..par la société "DYNAMIT NOBEL" | 4,000 g |
| Eau q.s.p. | 100,000 g |

Cette crème est appliquée 1 fois par jour, elle aide à lutter contre le vieillissement qu'il soit photoinduit ou chronologique.

## Revendications

1. Composés hétérocycliques aromatiques à chaîne latérale aromatique, caractérisés par le fait qu'ils répondent a la formule générale suivante: 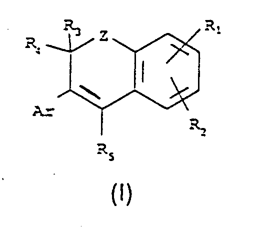 dans laquelle:
* Z représente un radical divalent choisi parmi les radicaux -O-, -S- ou -Nr'-,
* Ar représente soit le radical de formule (II) suivante : 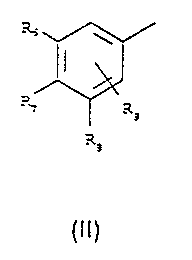 soit le radical de formule (III) suivante : 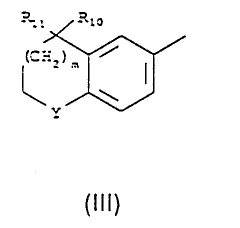 avec m est égal à 0 ou 1
* R₁ représente
(i) un atome d'hydrogène,
(ii) le radical -CH₃,
(iii) le radical -(CH₂)ₚ-O-R₁₂,
(iv) un radical -OR₁₂,
(v) un radical 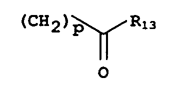
(vi) un radical -S(O)ₜR₁₄,
R₁₂, R₁₃, R₁₄, p et t ayant les significations données ci-après,
* R₂ représente un atome d'hydrogène, un atome d'halogène, un radical alkyle inférieur de C1-C6 ou le radical -OR₁₂,
R₁₂ ayant la signification donnée ci-après,
* R₃ et R₄, identiques ou différents, représentent un atome d'halogène, un atome d'hydrogène, un radical alkyle inférieur de C1-C6 ou un radical OR₁₂,
R₁₂ ayant la signification donnée ci-après,
* R₅ représente un atome d'halogène, un atome d'hydrogène, un radical alkyle inférieur de C1-C6 ou un radical OR₁₅,
R₁₅ ayant la signification donnée ci-après,
* R₆, R₇, R₈, R₉, identiques ou différents, sont choisis parmi:
- un atome d'hydrogène,
- un atome d'halogène,
- un radical alkyle,
- un radical cycloalkyle,
- un radical -(Z₁)ₙ-(CH₂)_{q}-CO-R₁₃,
- un radical -Z₂-R₁₂,
avec au moins deux des radicaux R₆, R₇, R₈ et R₉ différents de l'atome d'hydrogène, et au moins un des radicaux R₆, R₇, R₈ et R₉ représentant un radical alkyle ou un radical cycloalkyle, et R6 différent d'un radical méthyle quand R9 en position para par rapport à R6 est un radical hydroxy,
Z₁, Z₂, R₁₂, R₁₃, n, q ayant les significations données ci-après,
* R₁₀ et R₁₁ représentent des radicaux alkyles inférieur de C1-C6,
* Y représente un radical divalent choisi parmi les radicaux : -C(R₁₁)₂-, -O-, -S-, -Nr'-, -CH(OH)-, -CO-, -SO- et -SO₂-,
R₁₁ étant tel que défini ci-dessus,
r' ayant la signification donnée ci-après,
étant entendu que :
- R₁, R₂, R₃, R₄ et R₅ ne représentent pas simultanément l'atome d'hydrogène,
- R₁₂ représente un atome d'hydrogène, un radical alkyle inférieur de C1-C6, un radical aryle, un radical aralkyle, un radical polyhydroxyalkyle ou un radical polyether, un radical acyle inférieur de C1-C6,
- R₁₃ représente :
(a) un atome d'hydrogène,
(b) un radical 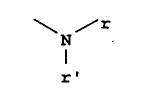 r et r' ayant les significations données ci-après,
(c) un radical -OR₁₄
R₁₄ ayant la signification donnée ci-après
- R₁₄ représente un atome d'hydrogène; un radical alkyle; un radical mono ou polyhydroxyalkyle; un radical aryle ou aralkyle, éventuellement substitué par un ou plusieurs atomes d'halogène, une fonction hydroxyle ou une fonction nitro, ou un groupe méthoxy; ou un reste de sucre ou d'aminoacide,
- R₁₅ représente un atome d'hydrogène, un radical alkyle inférieur de C1-C6, un radical aryle, un radical aralkyle ou un radical polyether,
- r et r', identiques ou différents, représentent un atome d'hydrogène; un radical alkyle inférieur de C1-C6; ou un radical aryle, éventuellement substitué par un ou plusieurs atomes d'halogène, une fonction hydroxyle ou une fonction nitro, ou un groupe méthoxy; ou un reste d'amino acide ou de sucre; ou encore, pris ensemble, forment un hétérocycle,
- Z₁ représente O, S ou Nr',
- Z₂ représente O ou S,
- n est égal à 0 ou 1,
- p est égal à 0, 1, 2 ou 3
- q est un entier prenant une valeur de 0 à 10,
- t est égal à 0, 1, 2 ou 3,
et les isomères optiques et géométriques desdits composés de formule (I), ainsi que leurs sels .

2. Composés selon la revendication 1, caractérisés par le fait qu'ils se présentent sous forme de sels d'un métal alcalin ou alcalino-terreux ou encore de zinc ou d'une amine organique.

3. Composés selon l'une des revendications précédentes, caractérisés par le fait que le radical alkyle inférieur est choisi dans le groupe constitué par les radicaux méthyle, éthyle, isopropyle, butyle et tertiobutyle.

4. Composés selon l'une quelconque des revendications précédentes, caractérisés par le fait que le radical acyle inférieur est un radical acétyle, propionyle ou pivaloyle.

5. Composés selon l'une quelconque des revendications précédentes, caractérisés par le fait que le radical cycloalkyle est un alcane cyclique ou polycyclique contenant de 1 à 10 atomes de carbone, éventuellement substitué par des atomes d' halogène ou des radicaux hydroxyle et de préférence les radicaux adamantyle ou 1-méthylcyclohéxyle.

6. Composés selon l'une quelconque des revendications précédentes, caractérisés par le fait que le radical polyéther est choisi parmi les radicaux méthoxyméthyl éther, méthoxyéthoxyméthyl éther ou méthylthyométhyl éther.

7. Composés selon l'une quelconque des revendications précédentes, caractérisés par le fait que le radical polyhydroxyalkyle est choisi dans le groupe constitué par le radical 2,3-dihydroxypropyle, 2,3,4-trihydroxybutyle, 2,3,4,5-tétrahydroxypentyle et le reste du pentaérythritol.

8. Composés selon l'une quelconque des revendications précédentes, caractérisés par le fait que le radical aryle éventuellement substitué est un radical phényle éventuellement substitué par au moins un atome d'halogène, un hydroxyle ou une fonction nitro.

9. Composés selon l'une quelconque des revendications précédentes, caractérisés par le fait que le radical aralkyle éventuellement substitué est le radical benzyle ou phénéthyle éventuellement substitué par au moins un atome d'halogène, un hydroxyle ou une fonction nitro.

10. Composés selon l'une quelconque des revendications précédentes, caractérisés par le fait que les restes d'aminoacide sont choisis dans le groupe constitué par les restes dérivant de la lysine, de la glycine, ou de l'acide aspartique.

11. Composés selon l'une quelconque des revendications précédentes, caractérisés par le fait que le reste de sucre est choisi dans le groupe constitué par les restes dérivant du glucose, du galactose, du mannose ou de l'acide glucuronique.

12. Composés selon l'une quelconque des revendications précédentes, caractérisés par le fait que l'hétérocycle est choisi dans le groupe constitué par un radical pipérino, morpholino, pyrrolidino ou pipérazino, éventuellement substitué en position 4 par un radical alkyle en C₁-C₆ ou mono ou polyhydroxyalkyle.

13. Composés selon l'une quelconque des revendications précédentes, caractérisés en ce que les composés de formule (I) sont ceux pour lesquels l'une au moins, et de préférence toutes, les conditions ci-dessous sont respectées:
- R1 est un radical -(CH₂)ₚ-CO-O-R₁₄
- R₂, R₃, R₄, R₅, R₆ et R₉ sont des atomes d'hydrogène
- R7 est un radical cycloalkyle
- R₈ est un radical -OR₁₂
- Z est un atome d' oxygène
- Y est un radical C(R₁₁)₂.

14. Composés selon l'une quelconque des revendications précédentes, caractérisés par le fait qu'ils sont choisis dans le groupe constitué par:
- Acide 3-[(3-(1-adamantyl)-4-méthoxyphényl)-2H-1-benzopyran-7-yl] carboxylique
- 3-[(3-(1-adamanthyl)-4-méthoxyphényl)-2H-1-benzopyran-7-yl] carboxylate de méthyle
- 3-[(3-(1-adamanthyl)-4-hydroxyphényl)-2H-1-benzopyran-7-yl] carboxylate de méthyle
- Acide 3-[(3-(1- adamantyl)-4-hydroxyphényl)-2H-1-benzopyran-7-yl] carboxylique
- Acide 3-[(3- (1-adamantyl)-4-méthoxyphényl)-2H-1-benzopyran-6-yl] carboxylique
- 3-[(3-(1-adamantyl)-4-méthoxyphényl)-2H-1-benzopyran-6-yl] carboxylate de méthyle
- Acide 3-[(3-(1- adamantyl)-4-hydroxyphényl)-2H-1-benzopyran-6-yl] carboxylique
- Acide 3-[(5,6,7,8-tétrahydro-5,5,8,8-tétraméthyl -2-naphtyl)-2H-1-benzopyran-7-yl] carboxylique
- 3-[(5,6,7,8-tétrahydro-5,5,8,8-tétraméthyl-2-naphtyl)-2H-1-benzopyran-7-yl] carboxylate de méthyle
- Acide 3-[(5,6,7,8-tétrahydro-5,5,8,8-tétraméthyl-2-naphtyl)-2H-1-benzopyran-6-yl] carboxylique
- 3-[(5,6,7,8-tétrahydro-5,5,8,8-tétraméthyl-2-naphtyl)-2H-1-benzopyran-6-yl] carboxylate de méthyle
- 3-[(3,5-Di-tert-butyl-4-méthoxyphényl)-2H-1-benzopyran-6-yl] carboxylate de méthyle
- Acide 3-[(3,5-Di-tert-butyl-4-méthoxyphényl)-2H-1-benzopyran-6-yl] carboxylique
- Acide 3-[(3,5-Di-tert-butyl-4-hydroxyphényl)-2H-1-benzopyran-6-yl] carboxylique
- 3-[(3,5-Di-tert-butyl-4-méthoxyphényl)-2H-1-benzopyran-7-yl] carboxylate de méthyle
- 3-[(3,5-Di-tert-butyl-4-méthoxyphényl)-2H-1-benzopyran-6-yl] carboxylate de méthyle
- Acide 3-[(3,5-Di-tert-butyl-4-hydroxyphényl) -2H-1-benzopyran-7-yl] carboxylique
- Acide 3-[(3,5-Di-tert-butyl-4-hydroxyphényl)-2H-1-benzopyran-7-yl] carboxylique
- 3-[(4-(1-adamantyl)-3-méthoxyphényl)-2H-1benzopyran]-6-carboxylate de méthyle
- acide 3-[(4-(1-adamantyl)-3-méthoxyphényl)-2H-1benzopyran]-6-carboxylique
- 3-[(3-tert-butyl-4-hydroxy phenyl)-2H-1-benzopyran]-7-carboxylate de méthyle
- Acide-3-[(3-tert-butyl-4-hydroxy phenyl)-2H-1-benzopyran]-7- carboxylique
- 3-[(3-(1-adamantyl)-4-méthoxyethoxyméthoxyphényl)-2H-1-benzopyran]-7-carboxylate de méthyl
- Acide 3-[(3-(1-adamantyl)-4-méthoxyethoxyméthoxyphényl)-2H-1-benzopyran]-7-carboxylique
- 3-[(5,6,7,8-tétrahydro-5,5,8,8-tétraméthyl-2-naphtyl)-2H-1-benzopyran]-7-méthyl alcohol
- 3-[(5,6,7,8-tétrahydro-5,5,8,8-tétraméthyl-2-naphtyl)-2H-1-benzopyran]-7-carboxaldéhyde
- 3-[(3,5-Di-tert-butyl-4-hydroxy phenyl)-2 H-I -benzopyran]-7-carboxanilide
- Morpholide de l'acide 3-[(5,6,7,8-tétrahydro-5,5,8,8-tétraméthylnaphtyl)-2H-1-benzopyran]-7-carboxylique
- Morpholide de l'acide 3-[(5,6,7,8-tétrahydro-5,5,8,8-tétraméthylnaphtyl)-2H-1-benzopyran]-7-glyoxylique
- N-butyl-3-[(5,6,7,8-tétrahydro-5,5,8,8-tétraméthylnaphtyl)-2H-1-benzopyran]-7-carboxamide.

15. Composés selon l'une quelconque des revendications précédentes pour une utilisation comme médicament.

16. Composés selon la revendication 15 pour une utilisation comme médicament destiné au traitement des affections dermatologiques, rhumatismales, respiratoires, cardiovasculaires et ophtalmologiques.

17. Composition pharmaceutique, caractérisée par le fait qu'elle contient dans un véhicule approprié, pour une administration par voie entérale, parentérale, topique ou oculaire, au moins un composé de formule (I) selon l'une quelconque des revendications 1 à 14.

18. Composition selon la revendication 17, caractérisée par le fait qu'elle contient de 0,001 à environ 5 % en poids d'un composé de formule (I).

19. Composition cosmétique pour l'hygiène corporelle et capillaire, caractérisée par le fait qu'elle contient, dans un véhicule cosmétique approprié, au moins un composé de formule (I) selon l'une quelconque des revendications 1 à 14.

20. Composition cosmétique selon la revendication 19, caractérisée par le fait qu'elle contient le composé de formule (I) à une concentration comprise entre 0,001 et 3 % en poids.

## Claims

1. Aromatic heterocyclic compounds containing an aromatic side chain, characterized in that they correspond to the following general formula: 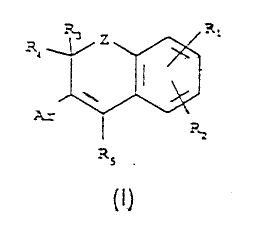 in which:
* Z represents a divalent radical chosen from the radicals -O-, -S- and -Nr'-,
* Ar represents either the radical of formula (II) below: 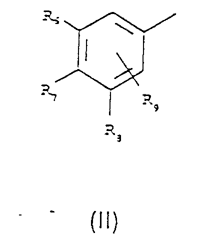 or the radical of formula (III) below: 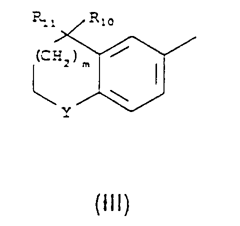 where m is equal to 0 or 1
* R₁ represents
(i) a hydrogen atom,
(ii) a -CH₃ radical,
(iii) the radical -(CH₂)ₚ-O-R₁₂,
(iv) a radical -OR₁₂,
(v) a radical 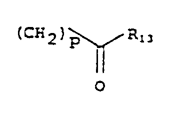
(vi) a radical -S(O)ₜR₁₄,
R₁₂, R₁₃, R₁₄, p and t having the meanings given below,
* R₂ represents a hydrogen atom, a halogen atom, a lower (C₁-C₆)alkyl radical or the radical -OR₁₂,
R₁₂ having the meaning given below,
* R₃ and R₄, which may be identical or different, represent a halogen atom, a hydrogen atom, a lower (C₁-C₆)alkyl radical or a radical OR₁₂,
R₁₂ having the meaning given below,
* R₅ represents a halogen atom, a hydrogen atom, a lower (C₁-C₆)alkyl radical or a radical OR₁₅,
R₁₅ having the meaning given below,
* R₆, R₇, R₈ and R₉, which may be identical or different, are chosen from:
- a hydrogen atom,
- a halogen atom,
- an alkyl radical,
- a cycloalkyl radical,
- a radical -(Z₁)ₙ-(CH₂)_{q}-CO-R₁₃,
- a radical -Z₂-R₁₂,
with at least two of the radicals R₆, R₇, R₈ and R₉ being other than a hydrogen atom, and at least one of the radicals R₆, R₇, R₈ and R₉ representing an alkyl radical or a cycloalkyl radical, and R₆ being other than a methyl radical when R₉, in the para position relative to R₆, is a hydroxyl radical,
Z₁, Z₂, R₁₂, R₁₃, n and q having the meanings given below,
* R₁₀ and R₁₁ represent lower (C₁-C₆)alkyl radicals,
* Y represents a divalent radical chosen from the radicals: -C(R₁₁)₂-, -O-, -S-, -Nr'-, -CH(OH)-, -CO-, -SO- and -SO₂-,
R₁₁ being as defined above,
r' having the meaning given below,
it being understood that:
- R₁, R₂, R₃, R₄ and R₅ do not simultaneously represent a hydrogen atom,
- R₁₂ represents a hydrogen atom, a lower (C₁-C₆)alkyl radical, an aryl radical, an aralkyl radical, a polyhydroxyalkyl radical, a polyether radical or a lower (C₁-C₆)acyl radical,
- R₁₃ represents:
(a) a hydrogen atom,
(b) a radical 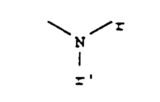 r and r' having the meanings given below,
(c) a radical -OR₁₄
R₁₄ having the meaning given below
- R₁₄ represents a hydrogen atom, an alkyl radical, a mono- or polyhydroxyalkyl radical, an aryl or aralkyl radical, optionally substituted with one or more halogen atoms, a hydroxyl function or a nitro function, or a methoxy group; or a sugar or amino acid residue,
- R₁₅ represents a hydrogen atom, a lower (C₁-C₆)alkyl radical, an aryl radical, an aralkyl radical or a polyether radical,
- r and r', which may be identical or different, represent a hydrogen atom; a lower (C₁-C₆)alkyl radical; or an aryl radical, which is optionally substituted with one or more halogen atoms, a hydroxyl function or a nitro function, or a methoxy group; or an amino acid or sugar residue; or alternatively, taken together, form a heterocycle,
- Z₁ represents O, S or Nr',
- Z₂ represents O or S,
- n is equal to 0 or 1,
- p is equal to 0, 1, 2 or 3
- q is an integer taking a value from 0 to 10,
- t is equal to 0, 1, 2 or 3,
and the optical and geometric isomers of the said compounds of formula (I), as well as the salts thereof.

2. Compounds according to Claim 1, characterized in that they are in the form of salts of an alkali metal or alkaline-earth metal or alternatively of zinc or of an organic amine.

3. Compounds according to either of the preceding claims, characterized in that the lower alkyl radical is chosen from the group consisting of the methyl, ethyl, isopropyl, butyl and tert-butyl radicals.

4. Compounds according to any one of the preceding claims, characterized in that the lower acyl radical is an acetyl, propionyl or pivaloyl radical.

5. Compounds according to any one of the preceding claims, characterized in that the cycloalkyl radical is a cyclic or polycyclic alkane containing from 1 to 10 carbon atoms, optionally substituted with halogen atoms or hydroxyl radicals and preferably adamantyl or 1-methylcyclohexyl radicals.

6. Compounds according to any one of the preceding claims, characterized in that the polyether radical is chosen from the methoxymethyl ether, methoxyethoxymethyl ether and methylthiomethyl ether radicals.

7. Compounds according to any one of the preceding claims, characterized in that the polyhydroxyalkyl radical is chosen from the group consisting of the 2,3-dihydroxypropyl, 2,3,4-trihydrobutyl and 2,3,4,5-tetrahydroxypentyl radicals and the pentaerythritol residue.

8. Compounds according to any one of the preceding claims, characterized in that the optionally substituted aryl radical is a phenyl radical optionally substituted with at least one halogen atom, a hydroxyl or a nitro function.

9. Compounds according to any one of the preceding claims, characterized in that the optionally substituted aralkyl radical is the benzyl or phenethyl radical optionally substituted with at least one halogen atom, a hydroxyl or a nitro function.

10. Compounds according to any one of the preceding claims, characterized in that the amino acid residues are chosen from the group consisting of residues derived from lysine, glycine or aspartic acid.

11. Compounds according to any one of the preceding claims, characterized in that the sugar residue is taken from the group consisting of residues derived from glucose, galactose, mannose or glucuronic acid.

12. Compounds according to any one of the preceding claims, characterized in that the heterocycle is chosen from the group consisting of a piperidino, morpholino, pyrrolidino or piperazino radical, optionally substituted in the 4-position with a (C₁-C₆)alkyl radical or mono- or polyhydroxyalkyl radical.

13. Compounds according to any one of the preceding claims, characterized in that the compounds of formula (I) are those for which at least one, and preferably all, of the conditions below are satisfied:
- R₁ is a radical -(CH₂)ₚ-CO-O-R₁₄
- R₂, R₃, R₄, R₅, R₆ and R₉ are hydrogen atoms
- R₇ is a cycloalkyl radical
- R₈ is a radical -OR₁₂
- Z is an oxygen atom
- Y is a radical C(R₁₁)₂.

14. Compounds according to any one of the preceding claims, characterized in that they are chosen from the group consisting of:
- 3-[(3-(1-Adamantyl)-4-methoxyphenyl)-2H-1-benzopyran-7-yl]carboxylic acid
- Methyl 3-[(3-(1-adamantyl)-4-methoxyphenyl)-2H-1-benzopyran-7-yl]carboxylate
- Methyl 3-[(3-(1-adamantyl)-4-hydroxyphenyl)-2H-1-benzopyran-7-yl]carboxylate
- 3-[(3-(1-Adamantyl)-4-hydroxyphenyl)-2H-1-benzopyran-7-yl]carboxylic acid
- 3-[(3-(1-Adamantyl)-4-methoxyphenyl)-2H-1-benzopyran-6-yl]carboxylic acid
- Methyl 3-[(3-(1-adamantyl)-4-methoxyphenyl)-2H-1-benzopyran-6-yl]carboxylate
- 3-[(3-(1-Adamantyl)-4-hydroxyphenyl)-2H-1-benzopyran-6-yl]carboxylic acid
- 3-[(5,6,7,8-Tetrahydro-5,5,8,8-tetramethyl-2-naphthyl)-2H-1-benzopyran-7-yl]carboxylic acid
- Methyl 3-[(5,6,7,8-tetrahydro-5,5,8,8-tetramethyl-2-naphthyl)-2H-1-benzopyran-7-yl]carboxylate
- 3-[(5,6,7,8-Tetrahydro-5,5,8,8-tetramethyl-2-naphthyl)-2H-1-benzopyran-6-yl]carboxylic acid
- Methyl 3-[(5,6,7,8-tetrahydro-5,5,8,8-tetramethyl-2-naphthyl)-2H-1-benzopyran-6-yl]carboxylate
- Methyl 3-[(3,5-di-tert-butyl-4-methoxyphenyl)-2H-1-benzopyran-6-yl]carboxylate
- 3-[(3,5-Di-tert-butyl-4-methoxyphenyl)-2H-1-benzopyran-6-yl]carboxylic acid
- 3-[(3,5-Di-tert-butyl-4-hydroxyphenyl)-2H-1-benzopyran-6-yl]carboxylic acid
- Methyl 3-[(3,5-di-tert-butyl-4-methoxyphenyl)-2H-1-benzopyran-7-yl]carboxylate
- Methyl 3-[(3,5-di-tert-butyl-4-methoxyphenyl)-2H-1-benzopyran-6-yl]carboxylate
- 3-[(3,5-Di-tert-butyl-4-hydroxyphenyl)-2H-1-benzopyran-7-yl]carboxylic acid
- 3-[(3,5-Di-tert-butyl-4-hydroxyphenyl)-2H-1-benzopyran-7-yl]carboxylic acid
- Methyl 3-[(4-(1-adamantyl)-3-methoxyphenyl)-2H-1-benzopyran]-6-carboxylate
- 3-[(4-(1-Adamantyl)-3-methoxyphenyl)-2H-1-benzopyran]-6-carboxylic acid
- Methyl 3-[(3-tert-butyl-4-hydroxyphenyl)-2H-1-benzopyran]-7-carboxylate
- 3-[(3-tert-Butyl-4-hydroxyphenyl)-2H-1-benzopyran]-7-carboxylic acid
- Methyl 3-[(3-(1-adamantyl)-4-methoxyethoxymethoxyphenyl)-2H-1-benzopyran]-7-carboxylate
- 3-[(3-(1-Adamantyl)-4-methoxyethoxymethoxyphenyl)-2H-1-benzopyran]-7-carboxylic acid
- 3-[(5,6,7,8-Tetrahydro-5,5,8,8-tetramethyl-2-naphthyl)-2H-1-benzopyran]-7-methyl alcohol
- 3-[(5,6,7,8-Tetrahydro-5,8,8,8-tetramethyl-2-naphthyl)-2H-1-benzopyran]-7-carboxaldehyde
- 3-[(3,5-Di-tert-butyl-4-hydroxyphenyl)-2H-1-benzopyran]-7-carboxanilide
- 3-[(5,6,7,8-Tetrahydro-5,5,8,8-tetramethylnaphthyl)-2H-1-benzopyran]-7-carboxylic acid morpholide
- 3-[(5,6,7,8-Tetrahydro-5,5,8,8-tetramethylnaphthyl)-2H-1-benzopyran]-7-glyoxylic acid morpholide
- N-Butyl-3-[(5,6,7,8-tetrahydro-5,5,8,8-tetramethylnaphthyl)-2H-1-benzopyran]-7-carboxamide.

15. Compounds according to any one of the preceding claims, for use as medicaments.

16. Compounds according to Claim 15, for use as medicaments intended for the treatment of dermatological, rheumatic, respiratory, cardiovascular and ophthalmological complaints.

17. Pharmaceutical composition, characterized in that it contains, in a suitable vehicle, for administration via the enteral, parenteral, topical or ocular route, at least one compound of formula (I) according to any one of Claims 1 to 14.

18. Composition according to Claim 17, characterized in that it contains from 0.001 to about 5% by weight of a compound of formula (I).

19. Cosmetic composition for body and hair hygiene, characterized in that it contains, in a suitable cosmetic vehicle, at least one compound of formula (I) according to any one of Claims 1 to 14.

20. Cosmetic composition according to Claim 19, characterized in that it contains the compound of formula (I) at a concentration of between 0.001 and 3% by weight.

## Patentansprüche

1. Aromatische, heterocyclische Verbindungen mit aromatischer Seitenkette,
**dadurch gekennzeichnet, daß**
sie der folgenden allgemeinen Formel entsprechen: 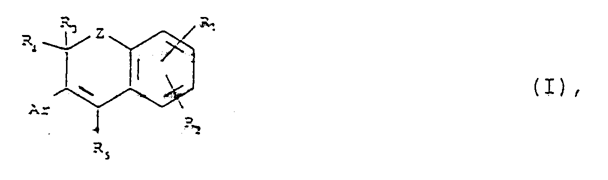 worin bedeuten:
* Z eine zweiwertige Gruppe, die unter den Gruppen -O-, -S- oder -Nr'- ausgewählt ist,
* Ar die Gruppe der folgenden Formel (II): 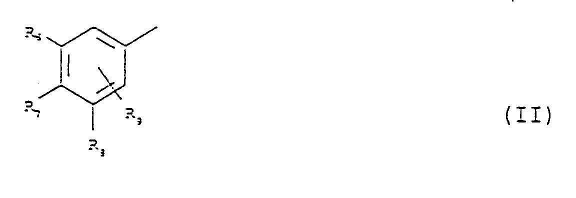 oder die Gruppe der folgenden Formel (III): 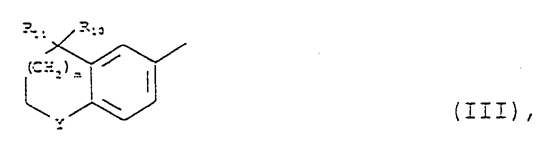 wobei m Null oder 1 bedeutet
* R₁
(i) Wasserstoff,
(ii) die Gruppe -CH₃
(iii) eine Gruppe -(CH₂)ₚ-O-R₁₂
(iv) eine Gruppe -OR₁₂
(v) eine Gruppe 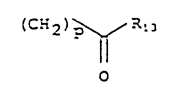
(vi) eine Gruppe -S(O)ₜR₁₄,
wobei R₁₂, R₁₃, R₁₄, p und t die nachstehend angegebenen Bedeutungen aufweisen,
* R₂ Wasserstoff, Halogen, eine niedere C₁₋₆-Alkylgruppe oder eine Gruppe -OR₁₂,
wobei R₁₂ die nachstehend angegebene Bedeutung aufweist,
* R₃ und R₄, die identisch oder voneinander verschieden sind, Halogen, Wasserstoff, eine niedere C₁₋₆-Alkylgruppe oder eine Gruppe OR₁₂,
wobei R₁₂ die nachstehend angegebene Bedeutung aufweist,
* R₅ Halogen, Wasserstoff, eine niedere C₁₋₆-Alkylgruppe oder eine Gruppe OR₁₅,
wobei R₁₅ die nachstehend angegebene Bedeutung aufweist,
* R₆, R₇, R₈ und R₉, die identisch oder voneinander verschieden sein können:
- Wasserstoff,
- Halogen,
- Alkyl,
- Cycloalkyl,
- -(Z₁)ₙ-(CH₂)_{q}-CO-R₁₃,
- -Z₂-R₁₂,
wobei mindestens zwei der Gruppen R₆, R₇, R₈ und R₉ von Wasserstoff verschieden sind und mindestens eine der Gruppen R₆, R₇, R₈ und R₉ eine Alkylgruppe oder eine Cycloalkylgruppe bedeutet und, wenn sich R₉ bezüglich R₆ in p-Stellung befindet und Hydroxy bedeutet, R₆ von Methyl verschieden ist,
wobei Z₁, Z₂, R₁₂, R₁₃, n und q die nachstehend angegebenen Bedeutungen aufweisen,
* R₁₀ und R₁₁ niedere C₁₋₆-Alkylgruppen,
* Y eine zweiwertige Gruppe, die unter den folgenden Gruppen ausgewählt ist: -C(R₁₁)₂-, -O-, -S-, -Nr', -CH(OH)-, -CO-, -SO- und -SO₂-, wobei R₁₁ die oben angegebene Bedeutung und r' die nachstehend angegebene Bedeutung aufweist,
mit der Maßgabe, daß:
- R₁, R₂, R₃, R₄ und R₅ nicht gleichzeitig Wasserstoff bedeuten,
- R₁₂ Wasserstoff, eine niedere C₁₋₆-Alkylgruppe, Aryl, Aralkyl, Polyhydroxyalkyl, eine Polyethergruppe oder eine niedere Acylgruppe bedeutet,
- R₁₃ bedeutet:
(a) Wasserstoff,
(b) eine Gruppe 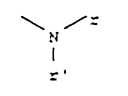 wobei r und r' die nachfolgend angegebenen Bedeutungen aufweisen,
(c) eine Gruppe -OR₁₄, wobei R₁₄ die nachfolgend angegebene Bedeutung aufweist,
- R₁₄ Wasserstoff, Alkyl, eine Mono- oder Polyhydroxyalkylgruppe; eine Aryl- oder Aralkylgruppe, die gegebenenfalls mit einem oder mehreren Halogenatomen, einer Hydroxygruppe, einer Nitrogruppe oder einer Methoxygruppe substituiert sind, einen Zuckerrest oder einen Aminosäurerest bedeutet,
- R₁₅ Wasserstoff, eine niedere Alkylgruppe, Aryl, Aralkyl oder eine Polyethergruppe bedeutet,
- r und r', die identisch oder voneinander verschieden sind, Wasserstoff, eine niedere Alkylgruppe, eine Arylgruppe, die gegebenenfalls mit einem oder mehreren Halogenatomen, Hydroxy, Nitro oder Methoxy substituiert ist, einen Aminosäurerest oder einen Zuckerrest bedeuten oder auch gemeinsam einen Heterocyclus bilden,
- Z₁ O, S oder Nr',
- Z₂ O oder S,
- n Null oder 1,
- p Null, 1, 2 oder 3,
- q Null oder eine ganze Zahl im Bereich von 1 bis 10,
- t Null, 1, 2 oder 3,
und die optischen und geometrischen Isomere der Verbindungen der Formel (I) sowie ihre Salze.

2. Verbindungen nach Anspruch 1, dadurch gekennzeichnet, daß sie in Form von Alkalisalzen, Erdalkalisalzen, Zinksalzen oder Salzen eines organischen Amins vorliegen.

3. Verbindungen nach einem der vorhergehenden Ansprüche, dadurch gekennzeichnet, daß die niedere Alkylgruppe unter den Gruppen Methyl, Ethyl, Isopropyl, Butyl und *tert*.-Butyl ausgewählt ist.

4. Verbindungen nach einem der vorhergehenden Ansprüche, dadurch gekennzeichnet, daß die niedere Acylgruppe eine Acetyl-, Propionyl- oder Pivaloylgruppe ist.

5. Verbindungen nach einem der vorhergehenden Ansprüche, dadurch gekennzeichnet, daß die Cycloalkylgruppe ein cyclisches oder polycyclisches Alkan mit 1 bis 10 Kohlenstoffatomen ist, das gegebenenfalls mit Halogenatomen oder Hydroxylgruppen und vorzugsweise den Gruppen Adamantyl oder 1-Methylcyclohexyl substituiert ist.

6. Verbindungen nach einem der vorhergehenden Ansprüche, dadurch gekennzeichnet, daß die Polyethergruppe unter den Gruppen Methoxymethylether, Methoxyethoxymethylether oder Methylthiomethylether ausgewählt ist.

7. Verbindungen nach einem der vorhergehenden Ansprüche, dadurch gekennzeichnet, daß die Polyhydroxyalkylgruppe unter den Gruppen 2,3-Dihydroxypropyl, 2,3,4-Trihydroxybutyl, 2,3,4,5-Tetrahydroxypentyl und der Pentaerythritgruppe ausgewählt ist.

8. Verbindungen nach einem der vorhergehenden Ansprüche, dadurch gekennzeichnet, daß die gegebenenfalls substituierte Arylgruppe eine Phenylgruppe ist, die gegebenenfalls mit mindestens einem Halogenatom, einer Hydroxygruppe oder einer Nitrogruppe substituiert ist.

9. Verbindungen nach einem der vorhergehenden Ansprüche, dadurch gekennzeichnet, daß die gegebenenfalls substituierte Aralkylgruppe eine Benzylgruppe oder Phenethylgruppe ist, die gegebenenfalls mit mindestens einem Halogenatom, einer Hydroxygruppe oder einer Nitrogruppe substituiert sind.

10. Verbindungen nach einem der vorhergehenden Ansprüche, dadurch gekennzeichnet, daß die Aminosäurereste unter den Resten ausgewählt sind, die von Lysin, Glycin oder Asparaginsäure abgeleitet sind.

11. Verbindungen nach einem der vorhergehenden Ansprüche, dadurch gekennzeichnet, daß der Zuckerrest unter den Resten ausgewählt ist, die von Glucose, Galactose, Mannose oder Glucuronsäure abgeleitet sind.

12. Verbindungen nach einem der vorhergehenden Ansprüche, dadurch gekennzeichnet, daß der Heterocyclus unter den Gruppen Piperidino, Morpholino, Pyrrolidino oder Piperazino ausgewählt ist, die gegebenenfalls in 4-Stellung mit einer C₁₋₆-Alkylgruppe oder einer Mono- oder Polyhydroxyalkylgruppe substituiert sind.

13. Verbindungen nach einem der vorhergehenden Ansprüche, dadurch gekennzeichnet, daß die Verbindungen der Formel (I) Verbindungen sind, die mindestens einer und vorzugsweise allen folgenden Bedingungen entsprechen:
- R₁ ist eine Gruppe -(CH₂)ₚ-CO-O-R₁₄
- R₂, R₃, R₄, R₅, R₆ und R₉ sind Wasserstoffatome
- R₄ ist eine Cycloalkylgruppe
- R₈ ist eine Gruppe -OR₁₂
- Z ist ein Sauerstoffatom
und
- Y ist eine Gruppe C(R₁₁)₂.

14. Verbindungen nach einem der vorhergehenden Ansprüche, dadurch gekennzeichnet, daß sie ausgewählt sind unter:
- [3-(3-(1-Adamantyl)-4-methoxyphenyl)-2H-1-benzopyran-7-yl]-carbonsäure
- Methyl-3-[(3-(1-adamantyl)-4-methoxyphenyl)-2H-1-benzopyran-7-yl]-carboxylat
- Methyl-3-[(3-(1-adamantyl)-4-hydroxyphenyl)-2H-1-benzopyran-7-yl]-carboxylat
- 3-[(3-(1-Adamantyl)-4-hydroxyphenyl)-2H-1-benzopyran-7-yl]-carbonsäure
- 3-[(3-(1-Adamantyl)-4-methoxyphenyl)-2H-1-benzopyran-6-yl]-carbonsäure
- Methyl-3-[(3-(1-adamantyl)-4-methoxyphenyl)-2H-1-benzopyran-6-yl]-carboxylat
- 3-[(3-(1-Adamantyl)-4-hydroxyphenyl)-2H-1-benzopyran-6-yl]-carbonsäure
- 3-[(5,6,7,8-Tetrahydro-5,5,8,8-tetramethyl-2-naphthyl)-2H-1-benzopyran-7-yl]-carbonsäure
- Methyl-3-[(5,6,7,8-tetrahydro-5,5,8,8-tetramethyl-2-naphthyl)-2H-1-benzopyran-7-yl]-carboxylat
- 3-[(5,6,7,8-Tetrahydro-5,5,8,8-tetramethyl-2-naphthyl)-2H-1-benzopyran-6-yl]-carbonsäure
- Methyl-3-[(5,6,7,8-tetrahydro-5,5,8,8-tetramethyl-2-naphthyl)-2H-1-benzopyran-6-yl]-carboxylat
- Methyl-3-[(3,5-Di-*tert*.-butyl-4-methoxyphenyl)-2H-1-benzopyran-6-yl]-carboxylat
- 3-[(3,5-Di-*tert*.-Butyl-4-methoxyphenyl)-2H-1-benzopyran-6-yl]-carbonsäure
- 3-[(3,5-Di-*tert*.-Butyl-4-hydroxyphenyl)-2H-1-benzopyran-6-yl]-carbonsäure
- Methyl-3-[(3,5-Di-*tert*.-butyl-4-methoxyphenyl)-2H-1-benzopyran-7-yl]-carboxylat
- Methyl-3-[(3,5-Di-*tert*.-butyl-4-methoxyphenyl)-2H-1-benzopyran-6-yl]-carboxylat
- 3-[(3,5-Di-*tert*.-Butyl-4-hydroxyphenyl)-2H-1-benzopyran-7-yl]-carbonsäure
- 3-[(3,5-Di-*tert*.-Butyl-4-hydroxyphenyl)-2H-1-benzopyran-7-yl]-carbonsäure
- Methyl-3-[(4-(1-adamantyl)-3-methoxyphenyl)-2H-1-benzopyran]-6-carboxylat
- 3-[(4-(1-Adamantyl)-3-methoxyphenyl)-2H-1-benzopyran]-6-carbonsäure
- Methyl-3-[(3-*tert*.-butyl-4-hydroxyphenyl)-2H-1-benzopyran]-7-carboxylat
- 3-[(3-*tert*.-Butyl-4-hydroxyphenyl)-2H-1-benzopyran]-7-carbonsäure
- Methyl-3-[(3-(1-adamantyl)-4-methoxyethoxymethoxyphenyl)-2H-1-benzopyran]-7-carboxylat
- 3-[(3-(1-Adamantyl)-4-methoxyethoxymethoxyphenyl)-2H-1-benzopyran]-7-carbonsäure
- 3-[(5,6,7,8-Tetrahydro-5,5,8,8-tetramethyl-2-naphthyl)-2H-1-benzopyran]-7-methylalkohol
- 3-[(5,6,7,8-Tetrahydro-5,5,8,8-tetramethyl-2-naphthyl)-2H-1-benzopyran]-7-carboxaldehyd
- 3-[(3,5-Di-*tert*.-Butyl-4-hydroxyphenyl)-2H-1-benzopyran]-7-carboxanilid
- Morpholid der 3-[(5,6,7,8-Tetrahydro-5,5,8,8-tetramethylnaphthyl)-2H-1-benzopyran]-7-carbonsäure
- Morpholid der 3-[(5,6,7,8-Tetrahydro-5,5,8,8-tetramethylnaphthyl)-2H-1-benzopyran]-7-glyoxylsäure
- N-Butyl-3-[(5,6,7,8-tetrahydro-5,5,8,8-tetramethylnaphthyl)-2H-1-benzopyran]-7-carboxamid.

15. Verbindungen nach einem der vorhergehenden Ansprüche zur Verwendung als Arzneimittel.

16. Verbindungen nach Anspruch 15 zur Verwendung als Arzneimittel, das zur Behandlung von dermatologischen Erkrankungen, rheumatischen Erkrankungen, Erkrankungen der Atemwege, kardiovaskulären Erkrankungen und ophthalmologischen Erkrankungen bestimmt ist.

17. Pharmazeutische Zusammensetzung, dadurch gekennzeichnet, daß sie in einem geeigneten Träger zur enteralen, parenteralen, topischen oder okularen Verabreichung mindestens eine Verbindung der Formel (I) nach einem der Ansprüche 1 bis 14 enthält.

18. Zusammensetzung nach Anspruch 17, dadurch gekennzeichnet, daß sie 0,001 bis etwa 5 Gew.-% einer Verbindung der Formel (I) enthält.

19. Kosmetische Zusammensetzung zur Körper- und Haarhygiene, dadurch gekennzeichnet, daß sie in einem kosmetisch geeigneten Träger mindestens eine Verbindung der Formel (I) nach einem der Ansprüche 1 bis 14 enthält.

20. Kosmetische Zusammensetzung nach Anspruch 19, dadurch gekennzeichnet, daß sie die Verbindung der Formel (I) in einer Konzentration von 0,001 bis 3 Gew.-% enthält.
